# EUROPEAN PATENT APPLICATION

(11) **EP 2 343 287 A1**
(43) Date of publication of application: **13.07.2011**
(21) Application number: 09382273.2
(22) Date of filing: 10.12.2009
(51) Int. Cl.: C07D 285/12, A61K 31/433

(54) **New 2-aminothiadiazole derivatives**

(71) Applicant: Almirall, S.A., 08022 Barcelona (ES)
(72) Inventor: Grima Poveda, Pedro Manuel, 08980 Sant Feliu de Llobregat (ES); Aguilar Izquierdo, Nuria, 08980 Sant Feliu de Llobregat (ES); Mir Cepeda, Marta, 08980 Sant Feliu de Llobregat (ES); Carrascal Riera, Marta, 08980 Sant Feliu de Llobregat (ES); Terricabras Belart, Emma, 08980 Sant Feliu de Llobregat (ES)
(74) Representative: Elzaburu Marquez, Alberto

(57) **Abstract**

The present invention relates to a compound of formula (I), or a pharmaceutically acceptable salt or N-oxide thereof: 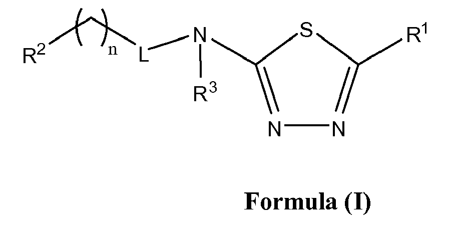 wherein:
• L represents a direct bond or a -S(O)₂- group,
• n is an integer having a value from 0 to 2,
• R1 represents a pyridyl group or an imidazo[1,2-a]pyridinyl group, wherein the pyridyl group is substituted with one or more substituents selected from halogen atoms, a hydroxy group, a C₁₋₄ alkyl group and a C₁₋₄ alkoxy group, or
• R¹ represents a group of formula: 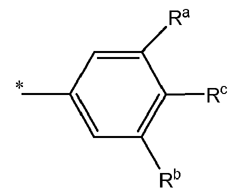 wherein:
o R^{a} and R^{b} independently represent a hydrogen atom, halogen atom or a linear or branched C₁₋₄ alkyl group,
o R^{c} represents a hydroxy group, a linear or branched C₁₋₄ alkyl group or C₁₋₄ alkoxy group wherein the alkyl and the alkoxy groups independently are optionally substituted with one or more substituents selected from hydroxy group, cyano group and-NR'R" groups and wherein
■ R' represents a hydrogen atom or a linear or branched C₁₋₄ alkyl group,
■ R" represents a hydrogen atom or a linear or branched C₁₋₄ alkyl group optionally substituted with a hydroxycarbonyl group; or
■ R' and R" together with the nitrogen atom to which they are attached form a 4 to 6 membered, saturated heterocyclic ring optionally substituted with a hydroxycarbonyl group.

• R² represents a pyridyl group, a C₃₋₆ cycloalkyl group or a phenyl group which is optionally substituted with one or more substituents selected from halogen atoms, cyano group, and C₁₋₄ alkoxy group; and
• R³ represents a C₃₋₆ cycloalkyl group, a C₃₋₆ cycloalkyl-C₁₋₂ alkyl group or a linear or branched C₂₋₄ alkyl group optionally substituted with one or more substituents selected from halogen atoms and C₁₋₂ alkoxy group.

## Description

The present invention relates to 2-aminothiadiazole derivatives, to processes for their preparation and to pharmaceutical compositions containing them. These compounds are potent agonists of S1 P1 receptors and thus, they are useful in the treatment, prevention or suppression of diseases and disorders known to be susceptible to improvement by sphingosine-1-phosphate receptors agonists (S1P1), such as autoimmune diseases, chronic immune and inflammatory diseases, transplant rejection, malignant neoplastic diseases, angiogenic-related disorders, pain, neurological diseases, viral and infectious diseases.

Sphingosine -1 phosphate (S1 P) is a pleiotropic lipid mediator that exhibits a broad spectrum of biological activities, including cell proliferation, survival, lymphocyte trafficking, cytoskeletal organization, and morphogenesis. S1 P is generated from endogenous sphingosine through phosphorylation by specific kinases, named sphingosine kinases 1 and 2. The levels of S1 P in biological fluids and tissues are tightly regulated by the balance between its synthesis by sphingosine kinases and its degradation by S1 P lyase. This tight control is important since an excessive production of S1 P has been associated to various pathological conditions, such as angiogenesis and vascular permeability changes in cancer, inflammation, myocardial infarction or transplant rejection.

Gene deletion studies and reverse pharmacology have provided evidence that most of the effects of S1 P are mediated via five G-protein coupled receptor subtypes, named S1 P1 to S1 P5 (Brinkmann, Pharmacology & therapeutics 115:84-105, 2007). The interest on this family of receptors increased following the discovery that they were the pharmacological target of FTY720. This compound, a synthetic analog of a natural product derived from the fungus *Isaria sinclairii,* exhibited a peculiar immunomodulatory potential in vivo. When administered in vivo, it caused lymphopenia, due to the sequestration of lymphocytes from the blood into the lymph nodes and Peyer's patches. The close structural similarity of FTY720 to sphingosine, together with the discovery of the formation of phosphorylated FTY720 in vivo (FTY720P) prompted to speculate that FTY720-P could be acting as a mimetic of S1 P. This proven to be the case and it was later on demonstrated that FTY-P binds 4 of the five known S1 P receptors, namely S1 P1, S1 P3, S1 P4 and S1 P5.

Expression analysis identified S1 P1 as the dominant S1 P receptor expressed on lymphocytes. Moreover, the transfer of S1P1-deficient T cells to normal mice led to the cells being sequestered in lymph nodes, as occurred with animals treated with fingolimod. These two facts strongly pointed out at S1P1 as the main receptor involved in the lymphopenic effect of FTY-P in vivo (Baumruker et al, Exp. Opin. Invest. Drugs 2007; 16(3): 283-289). FTY720 is currently in phase III trials for the treatment of relapsing-remitting multiple sclerosis. The drug is presumed to act by causing the retention of pathogenic lymphocytes in lymph nodes, thus preventing them to infiltrate the central nervous system (CNS).

In view of the physiological effects, several S1 P1 agonists have been recently disclosed for the treatment or prevention of autoimmune diseases, such as multiple sclerosis (WO2008000419, WO2008021532), rheumatoid arthritis or Crohn's disease (WO2007091501), chronic immune and inflammatory diseases such as asthma, transplant rejection (WO199400943), cancer (WO2003097028), lymphoid malignancies (WO2007143081), angiogenic-related disorders, pain (WO2004110421, WO2007089715) neurological diseases such as neurodegeneration (WO2005025553) or dementia (WO2005058295), cardiovascular diseases (WO2004010987),.

Autoimmune diseases include but are not limited to rheumatoid arthritis, multiple sclerosis, inflammatory bowel diseases such as Crohn's diseases and ulcerative colitis, psoriatic arthritis, thyroiditis such as Hashimoto's thyroiditis, type I diabetes; systemic lupus erythematosis and Sjögrn's syndrome.

Rejection transplanted organs such as kidney, liver, heart, lung, pancreas, cornea and skin; graft-versus-hist disease brought about by stem cell transplantation.

Immune and inflammatory diseases which may be prevented or treated include but are not limited to asthma, COPD, respiratory distress syndrome, acute or chronic pancreatitis and hepatitis; chronic sarcoidosis, contact dermatitis, atopic dermatitis, allergic rhinitis, allergic conjunctivitis, Behcet syndrome, inflammatory eye conditions such as conjunctivitis and uveitis.

Malignant neoplastic diseases that may be prevented or treated include but are not limited to solid cancer, tumor metastasis and lymphoid malignancies

Angiogenesis-related disorders that may be prevented or treated include but are not limited to hemangiomas, ocular neovascularization, macular degeneration or diabetic retinopathy.

Pain including neuropathic pain, that may be prevented or treated include but are not limited to prophylaxis or treatment of chronic pain, wherein chronic pain is selected from chronic muscular diseases such as back pain, pain during menstruation, pain during osteoarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, neuropathic pain e. g. after amputation,trigeminal neuralgia, migraine or post herpetic neuralgia.

Cardiovascular diseases which may be prevented or treated include but are not limited to chronic heart failure, congestive heart failure, arrhythmia or tachyarrythmia, unstable angina, acute myocardial infarction or complications from cardiac surgery or for improving heart energy efficiency or cardiac output.

Neurological diseases including neurodegeneration, dementia or brain degeneration that may be prevented or treated include but are not limited to neurological disorders including Parkinson's desease, Parkinsonian disorders, Huntington's disease, Alzheimer's disease, amyotrophic lateral sclerosis, spinal ischemia, ischemic stroke, spinal cord injury, cancer-related brain injury, and cancer-related spinal cord injury, Shy-Drager syndrome, progressive supranuclear palsy, Lewy body disease, stroke, cerebral infarction, multi-infarct dementia, and geriatric dementia,

Viral diseases which may be prevented or treated include but are not limited to HIV infection, hepatitis C and cytomegalovirus infection.

Infectious diseases which may be prevented or treated include but are not limited to pathogenic fungal diseases.

It has now been found that certain 2-aminothiadiazoles are novel and potent agonists of S1 P1 and can therefore be used in the treatment or prevention of these diseases.

Thus the present invention is directed to new 2-aminothiadiazole derivatives of formula (I) or pharmaceutically acceptable salts or N-oxides thereof 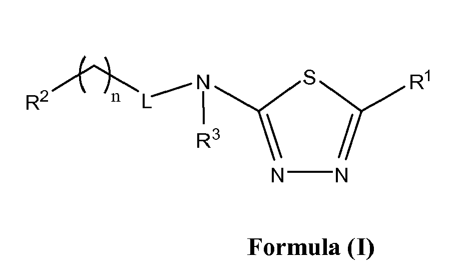 wherein:
- L represents a direct bond or a -S(O)₂- group,
- n is an integer having a value from 0 to 2,
- R¹ represents a pyridyl group or an imidazo[1,2-α]pyridinyl group, wherein the pyridyl group is substituted with one or more substituents selected from halogen atoms, a hydroxy group, a linear or branched C₁₋₄ alkyl group and a C₁₋₄ alkoxy group, or
- R¹ represents a group of formula: 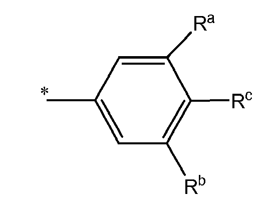 wherein:
   ○ R^{a} and R^{b} independently represent a hydrogen atom, a halogen atom or a linear or branched C₁₋₄ alkyl group,
   ○ R^{c} represents a hydroxy group, a linear or branched C₁₋₄ alkyl group or C₁₋₄ alkoxy group wherein the alkyl and the alkoxy groups independently are optionally substituted with one or more substituents selected from hydroxy group, a cyano group and a - NR'R" group and wherein
      ■ R' represents a hydrogen atom or a linear or branched C₁₋₄ alkyl group,
      ■ R" represents a hydrogen atom or a linear or branched C₁₋₄ alkyl group optionally substituted with a hydroxycarbonyl group; or
      ■ R' and R" together with the nitrogen atom to which they are attached form a 4 to 6 membered, saturated heterocyclic ring optionally substituted with a hydroxycarbonyl group.
- R² represents a pyridyl group, a C₃₋₆ cycloalkyl group or a phenyl group which is optionally substituted with one or more substituents selected from halogen atoms, cyano group, and C₁₋₄ alkoxy group;
- R³ represents a C₃₋₆ cycloalkyl group, a C₃₋₆ cycloalkyl-C₁₋₂ alkyl group or a linear or branched C₂₋₄ alkyl group optionally substituted with one or more substituents selected from halogen atoms and C₁₋₂ alkoxy group;

Further objectives of the present invention are to provide a method for preparing said compounds; pharmaceutical compositions comprising an effective amount of said compounds; compounds for use in the treatment of a human or animal body, in particular, for the treatment of pathological conditions or diseases susceptible to improvement by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases. The present invention further provides the use of the compounds in the manufacture of a medicament for the treatment of such pathological condition or diseases and methods of treatment of such pathological conditions or diseases susceptible to amelioration by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases comprising the administration of the compounds of the invention to a subject in need of treatment.

As used herein the term alkyl embraces linear or branched hydrocarbon radicals having 1 to 4 carbon atoms. Examples include methyl, ethyl, *n*-propyl, *i*-propyl, *n*-butyl, *i*-butyl and *ter*-butyl radicals.

As used herein the term alkoxy embraces linear or branched oxy-containing radicals each having 1 to 4 carbon atoms. Examples include methoxy, ethoxy, *n*-propoxy, *i-*propoxy, *n*-butoxy, and *tert*-butoxy radicals.

As used herein, the term cycloalkyl embraces saturated carbocyclic radicals and, unless otherwise specified, a cycloalkyl radical typically has from 3 to 6 carbon atoms, preferably from 3 to 4 carbon atoms.

Examples include cyclopropyl, cyclobutyl, cyclopentyl and cyclohexyl.

The 4 to 6-membered saturated heterocyclic ring which R' and R" may form together with the nitrogen to which they are attached is a saturated C₄-C₆ carbocyclic ring system in which one of the carbon atoms is replaced by N and optionally in which 1 further carbon atom is replaced by a heteroatom selected from N, O and S. Thus, said 4 to 6-membered saturated heterocyclic group contains, as heteroatoms, the N atom to which R' and R" are attached and 0 or 1 further N, O or S atoms.

Examples of 4 to 6-membered saturated heterocyclic radicals include azetidyl, piperidyl, pyrrolidyl, piperazinyl, morpholinyl and thiomorpholinyl. Azetidyl and piperidyl are preferred.

When R³ represents a C₃₋₆ cycloalkyl-C₁₋₂ alkyl group, it is to be understood that the C₁₋₂ alkyl portion is preferably bonded to the nitrogen atom of the molecule as depicted in formula (I).

As used herein, some of the atoms, radicals, moieties, chains or cycles present in the general structures of the invention are "optionally substituted". This means that these atoms, radicals, moieties, chains or cycles can be either unsubstituted or substituted in any posiition by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term halogen atom embraces chlorine, fluorine, bromine or iodine atoms typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

Typically, L represents a direct bond.

Typically, n has a value of 1 or 2, preferably 1.

Typically, R¹ represents a pyridyl group substituted with one or two substituents selected from halogen atoms, a methyl group and a methoxy groups, or R¹ represents a group of formula: 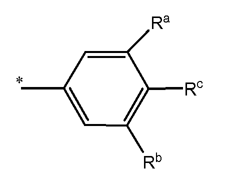 wherein:
○ R^{a} and R^{b} independently represent a hydrogen atom or a linear or branched C₁-₄ alkyl group,
○ R^{c} represents a hydroxy group, a linear or branched C₁₋₄ alkyl group or C₁₋₄ alkoxy group wherein the alkyl and the alkoxy groups independently are optionally substituted with one or more substituents selected from a hydroxy group, a cyano group and a -NR'R" groups wherein
   ■ R' represents a hydrogen atom or a methyl group,
   ■ R" represents a hydrogen atom or a linear or branched C₁₋₄ alkyl group optionally substituted with a hydroxycarbonyl group; or
   ■ R' and R" together with the nitrogen atom to which they are attached form a 4 to 6 membered, saturated heterocyclic ring substituted with a hydroxycarbonyl group.

Preferably, R¹ represents a pyridyl group substituted with one substituent selected from a methyl group and a methoxy group, or R¹ represents a group of formula: 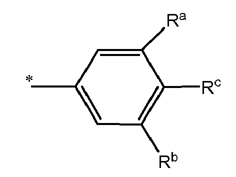 wherein:
○ both R^{a} and R^{b} represent a methyl group,
○ R^{c} represents a hydroxy group, a methoxy group or a C₂₋₄ alkoxy group substituted with one or two substituents selected from a hydroxy group and a -NR'R" groups wherein
   ■ R' represents a hydrogen atom or a methyl group,
   ■ R" represents a hydrogen atom, a C₁₋₂ alkyl group substituted with a hydroxycarbonyl group; or
   ■ R' and R" together with the nitrogen atom to which they are attached form a 4 to 6 membered, saturated heterocyclic ring substituted with a hydroxycarbonyl group.

Typically, R² represents a phenyl group which is substituted with one or two substituents selected from chlorine atoms, fluorine atoms, cyano group and methoxy group, preferably fluorine atoms, chlorine atoms and cyano groups.

Typically, R³ represents a linear or branched C₂₋₄ alkyl group optionally substituted with one or more substituents selected from a halogen atom and a C₁₋₂ alkoxy group.

Preferably, R³ represents a propyl or a butyl group, more preferably a butyl group.

Most preferably, L represents a direct bond, n has a value of 1, R¹ represents a pyridyl group substituted with one substituent selected from a methyl group and a methoxy group, or R¹ represents a group of formula: 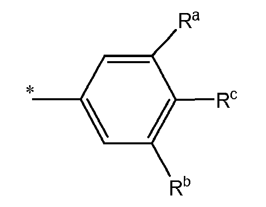 wherein:
○ both R^{a} and R^{b} represent a methyl group,
○ R^{c} represents a hydroxy group, a methoxy group or a C₂₋₄ alkoxy group substituted with one or two substituents selected from a hydroxy group, and a -NR'R" groups wherein
   ■ R' represents a hydrogen atom or a methyl group,
   ■ R" represents a hydrogen atom, a C₁₋₂ alkyl group substituted with a hydroxycarbonyl group; or
   ■ R' and R" together with the nitrogen atom to which they are attached form a 4 to 6 membered, saturated heterocyclic ring substituted with a hydroxycarbonyl group;

R² represents a phenyl group which is substituted with one or two substituents selected from chlorine atoms, fluorine atoms and a cyano group, and R³ represents a butyl group.

Typically, R¹ represents a pyridyl group or an imidazo[1,2-α]pyridinyl group, wherein the pyridyl group is substituted with one or more substituents selected from chlorine atoms, a hydroxy group, a methyl group and a methoxy group, or R¹ represents a group of formula: 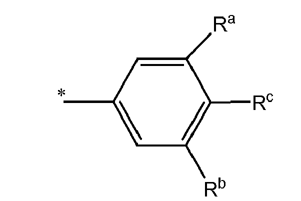 wherein:
○ R^{a} and R^{b} independently represent a hydrogen atom, a chlorine atom or a methyl group,
○ R^{c} represents a hydroxy group, a linear or branched C₁₋₄ alkyl group or C₁₋₄ alkoxy group wherein the alkyl and the alkoxy groups independently are optionally substituted with one or more substituents selected from a hydroxy group, a cyano group and a -NR'R" groups and wherein
   ■ R' represents a hydrogen atom or a methyl group,
   ■ R" represents a hydrogen atom, a C₁₋₂ alkyl group optionally substituted with a hydroxycarbonyl group; or
   ■ R' and R" together with the nitrogen atom to which they are attached form a 4 to 6 membered, saturated heterocyclic ring optionally substituted with a hydroxycarbonyl group.

R² represents a pyridyl group, a cyclobutyl group or a phenyl group which is optionally substituted with one or more substituents selected from chlorine atoms, fluorine atoms, a cyano group and a methoxy group; and R³ represents a cyclopentyl group, a cylopropylmethyl group, ethyl group, isopropyl group, a propyl group, n-butyl group, 4-trifluorobutyl group or a methoxyethyl group.

Particular individual compounds of the invention include:
N-butyl-N-(2-chlorobenzyl)-5-(4-methoxy-3,5-dimethylphenyl)-1,3,4-thiadiazol-2-amine
N-benzyl-N-butyl-5-(4-methoxy-3,5-dimethylphenyl)-1,3,4-thiadiazol-2-amine N-butyl-N-(2-fluorobenzyl)-5-(4-methoxy-3,5-dimethylphenyl)-1,3,4-thiadiazol-2-amine
N-butyl-N-(2,6-difluorobenzyl)-5-(4-methoxy-3,5-dimethylphenyl)-1,3,4-thiadiazol-2-amine
N-butyl-N-(2,4-difluorobenzyl)-5-(4-methoxy-3,5-dimethylphenyl)-1,3,4-thiadiazol-2-amine
N-butyl-N-(2,6-dichlorobenzyl)-5-(4-methoxy-3,5-dimethylphenyl)-1,3,4-thiadiazol-2-amine
2-({butyl[5-(4-methoxy-3,5-dimethylphenyl)-1,3,4-thiadiazol-2-yl]amino}methyl)-benzonitrile
N-butyl-N-(3-methoxybenzyl)-5-(4-methoxy-3,5-dimethylphenyl)-1,3,4-thiadiazol-2-amine
N-(2-chlorobenzyl)-N-(cyclopropylmethyl)-5-(4-methoxy-3,5-dimethylphenyl)-1,3,4-thiadiazol-2-amine
N-(2-chlorobenzyl)-5-(4-methoxy-3,5-dimethylphenyl)-N-(2-methoxyethyl)-1,3,4-thiadiazol-2-amine
N-(2-chlorobenzyl)-5-(4-methoxy-3,5-dimethylphenyl)-N-propyl-1,3,4-thiadiazol-2-amine
N-(2-chlorobenzyl)-5-(4-methoxy-3,5-dimethylphenyl)-N-(4,4,4-trifluorobutyl)-1,3,4-thiadiazol-2-amine
N-(2-chlorobenzyl)-N-cyclopentyl-5-(4-methoxy-3,5-dimethylphenyl)-1,3,4-thiadiazol-2-amine
N-(2-chlorobenzyl)-N-isopropyl-5-(4-methoxy-3,5-dimethylphenyl)-1,3,4-thiadiazol-2-amine
N-butyl-N-(2-fluorobenzyl)-5-(2-methylpyridin-4-yl)-1,3,4-thiadiazol-2-amine
N-butyl-N-(2,6-dichlorobenzyl)-5-(2-methylpyridin-4-yl)-1,3,4-thiadiazol-2-amine
N-butyl-N-(cyclobutylmethyl)-5-(2-methylpyridin-4-yl)-1,3,4-thiadiazol-2-amine
N-butyl-5-(2-methylpyridin-4-yl)-N-(2-phenylethyl)-1,3,4-thiadiazol-2-amine
5-(4-methoxy-3,5-dimethylphenyl)-N-propyl-N-(pyridin-4-ylmethyl)-1,3,4-thiadiazol-2-amine
N-butyl-N-(2,6-dichlorobenzyl)-5-imidazo[1,2-a]pyridin-6-yl-1,3,4-thiadiazol-2-amine
N-butyl-N-(2-chlorobenzyl)-5-imidazo[1,2-a]pyridin-6-yl-1,3,4-thiadiazol-2-amine 1-(4-{5-[butyl(2-chlorobenzyl)amino]-1,3,4-thiadiazol-2-yl}benzyl)azetidine-3-carboxylic acid
1-(4-{5-[butyl(2,6-dichlorobenzyl)amino]-1,3,4-thiadiazol-2-yl}benzyl)azetidine-3-carboxylic acid
N-butyl-5-(2-chloro-6-methoxypyridin-4-yl)-N-(2-fluorobenzyl)-1,3,4-thiadiazol-2-amine
N-butyl-N-(2-fluorobenzyl)-5-(2-methoxypyridin-4-yl)-1,3,4-thiadiazol-2-amine
N-butyl-N-(2,6-dichlorobenzyl)-5-(6-methoxypyridin-3-yl)-1,3,4-thiadiazol-2-amine
4-{5-[butyl(2-chlorobenzyl)amino]-1,3,4-thiadiazol-2-yl]-2,6-dimethylphenol N-butyl-N-(2,6-dichlorobenzyl)-5-imidazo[1,2-a]pyridin-7-yl-1,3,4-thiadiazol-2-amine
(2R)-3-(4-{5-[butyl(2-chlorobenzyl)amino]-1,3,4-thiadiazol-2-yl}-2,6-dimethylphenoxy)-propane-1,2-diol
(4-{5-[butyl(2,6-dichlorobenzyl)amino]-1,3,4-thiadiazol-2-yl}phenyl)acetonitrile (2S)-3-(4-{5-[butyl(2-chlorobenzyl)amino]-1,3,4-thiadiazol-2-yl}-2,6-dimethylphenoxy)-propane-1,2-diol
4-{5-[butyl(2-fluorobenzyl)amino]-1,3,4-thiadiazol-2-yl}pyridin-2-ol N-benzyl-N-butyl-5-(3-chloro-4-methoxyphenyl)-1,3,4-thiadiazol-2-amine
1-amino-3-(4-{5-[butyl(2-chlorobenzyl)amino]-1,3,4-thiadiazol-2-yl}-2,6-dimethyl-phenoxy)propan-2-ol
1-[2-(4-{5-[butyl(2-fluorobenzyl)amino]-1,3,4-thiadiazol-2-yl}-2,6-dimethylphenoxy)-ethyl]azetidine-3-carboxylic acid
N-[2-(4-{5-[butyl(2-fluorobenzyl)amino]-1,3,4-thiadiazol-2-yl}-2,6-dimethylphenoxy)-ethyl]-beta-alanine
N-[2-(4-{5-[butyl(2-fluorobenzyl)amino]-1,3,4-thiadiazol-2-yl}-2,6-dimethylphenoxy)-ethyl]-N-methylglycine
1-[2-(4-{5-[butyl(2-fluorobenzyl)amino]-1,3,4-thiadiazol-2-yl}-2,6-dimethylphenoxy)-1-cyanoethyl]azetidine-3-carboxylic acid
1-[2-(4-{5-[butyl(2-fluorobenzyl)amino]-1,3,4-thiadiazol-2-yl}-2,6-dimethylphenoxy)-ethyl]piperidine-4-carboxylic acid
1-(4-{5-[ethyl(pyridin-2-yl)amino]-1,3,4-thiadiazol-2-yl}benzyl)azetidine-3-carboxylic acid
1-(4-{5-[butyl(pyridin-2-yl)amino]-1,3,4-thiadiazol-2-yl}benzyl)azetidine-3-carboxylic acid
N-butyl-N-[5-(4-methoxy-3,5-dimethylphenyl)-1,3,4-thiadiazol-2-yl]benzenesulfonamide
1-(4-{5-[(benzylsulfonyl)(butyl)amino]-1,3,4-thiadiazol-2-yl}benzyl)azetidine-3-carboxylic acid
1-(4-{5-[butyl(phenylsulfonyl)amino]-1,3,4-thiadiazol-2-yl}benzyl)azetidine-3-carboxylic acid
1-(4-{5-[(benzylsulfonyl)(ethyl)amino]-1,3,4-thiadiazol-2-yl}benzyl)azetidine-3-carboxylic acid

Of outstanding interest are:
N-butyl-N-(2-chlorobenzyl)-5-(4-methoxy-3,5-dimethylphenyl)-1,3,4-thiadiazol-2-amine
N-butyl-N-(2,6-dichlorobenzyl)-5-(4-methoxy-3,5-dimethylphenyl)-1,3,4-thiadiazol-2-amine
2-({butyl[5-(4-methoxy-3,5-dimethylphenyl)-1,3,4-thiadiazol-2-yl]amino}methyl)-benzonitrile
N-butyl-N-(2,6-dichlorobenzyl)-5-(2-methylpyridin-4-yl)-1,3,4-thiadiazol-2-amine
4-{5-[butyl(2-chlorobenzyl)amino]-1,3,4-thiadiazol-2-yl}-2,6-dimethylphenol
(2R)-3-(4-{5-[butyl(2-chlorobenzyl)amino]-1,3,4-thiadiazol-2-yl}-2,6-dimethyl-phenoxy)propane-1,2-diol
(2S)-3-(4-{5-[butyl(2-chlorobenzyl)amino]-1,3,4-thiadiazol-2-yl}-2,6-dimethyl-phenoxy)propane-1,2-diol
1-amino-3-(4-{5-[butyl(2-chlorobenzyl)amino]-1,3,4-thiadiazol-2-yl}-2,6-dimethyl-phenoxy)propan-2-ol
1-[2-(4-{5-[butyl(2-fluorobenzyl)amino]-1,3,4-thiadiazol-2-yl}-2,6-dimethylphenoxy)-ethyl]azetidine-3-carboxylic acid
N-[2-(4-{5-[butyl(2-fluorobenzyl)amino]-1,3,4-thiadiazol-2-yl}-2,6-dimethylphenoxy)-ethyl]-beta-alanine
N-[2-(4-{5-[butyl(2-fluorobenzyl)amino]-1,3,4-thiadiazol-2-yl}-2,6-dimethylphenoxy)-ethyl]-N-methylglycine
1-[2-(4-{5-[butyl(2-fluorobenzyl)amino]-1,3,4-thiadiazol-2-yl}-2,6-dimethylphenoxy)-ethyl]piperidine-4-carboxylic acid

Compounds of general formula (I) may be prepared following the synthetic scheme depicted in figure 1.

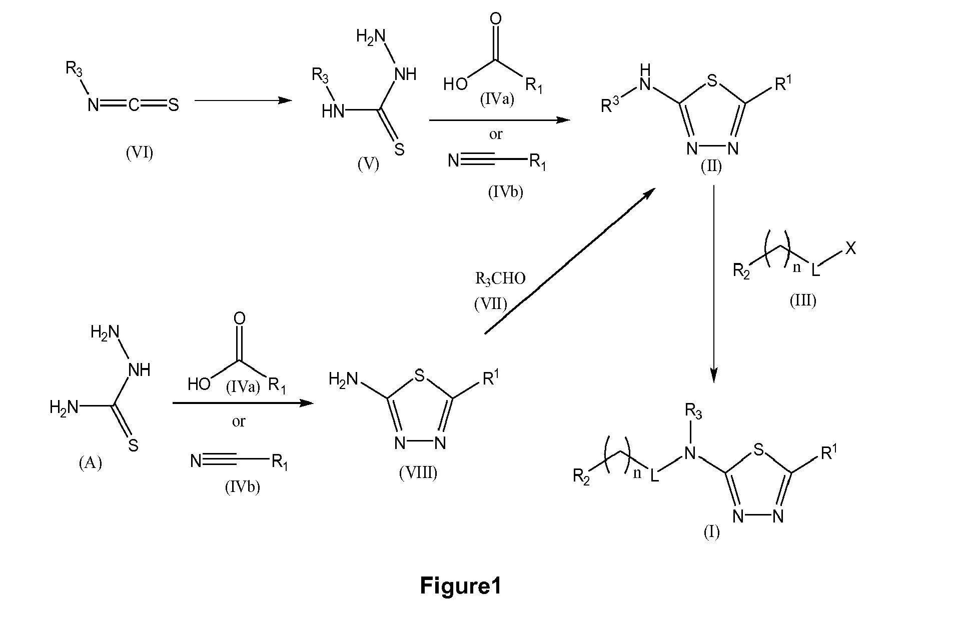

Compounds of general formula (I) may be prepared by the reaction of 1,3,4-thiadiazol-2-ylamine derivatives (II), wherein R¹ and R³ are as described above, with the corresponding alkylating agent (III), wherein L is a direct bond, R² is as described above and X is an halogen atom such as chlorine, bromine or iodide or a sulphonate such as mesylate, tosylate or triflate, in basic media such as sodium hydride in a solvent such as THF or DMF at a temperature from 0 to 150°C. Microwave may be used as an alternative heating procedure.

In the particular case in which n has a value of 0, the presence of a palladium catalyst such as palladium acetate and a phospine such as BINAP (2,2'-bis(diphenylphosphino)-1,1'-binaphthyl) or other palladium catalysts such as tetrakistriphenylphosphine palladium (0), tris(dibenzylideneacetone)palladium (0) or bis(tri-*orto*-tolylphosphine) palladium (II) chloride and a base such as soidum *tert-*butoxide or sodium bis(trimethylsilyl)amide in an solvent such as toluene or xylene must be required. The reaction may be performed from room temperature to the solvent boiling point.

Compounds of general formula (II) may be prepared by condensation of the compounds of formula (V) with the corresponding acid derivative (IVa) using POCl₃. Alternatively they may be prepared by condensation of compounds of formula (V) with the corresponding nitrile (IVb) in the presence of trifluoroacetic acid. Both reactions may be performed without a solvent or in a solvent such as dioxane or THF or dichloromethane at a temperature from 20 to 150°C.

Intermediates of formula (V) may be obtained by the reaction of hydrazine hydrate with the corresponding isothiocyanate (VI) in a solvent such as THF, methanol or ethanol and at a temperature from 0 to 40°C.

Alternatively, Intermediates of general formula (II) may be prepared by reductive amination of compounds of general formula (VIII) with the corresponding aldehyde (VII) in acid media such as acetic acid and in a protic solvent such as methanol or ethanol and with a reductive agent such as sodium borohydride, triacetoxybotohydride or sodium cyanoborohydride at a temperature from 0°C to the boiling point of the solvent. As an alternative to the acidic media a Lewis acid such as zinc chloride can be used.

Intermediates of general formula (VIII) may be prepared by condensation of hydrazinecarbothioamide of formula (A) with the corresponding acid derivative (IVa) using POCl₃ or with the corresponding nitrile derivative (IVb) in the presence of TFA. Both reactions may be performed without a solvent in a solvent such as dioxane or THF or dichloromethane at a temperature from 20 to 100°C.

In the particular case in where L is a -S(O)₂- group, the compounds of general formula (Ia) can be prepared according to the synthetic scheme depicted in figure 2.

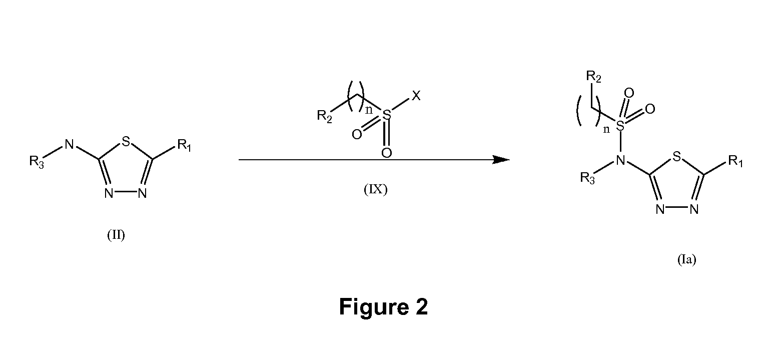

The compounds of formula (Ia) may be obtained by the reaction of 1,3,4-thiadiazol-2-amine derivatives (II), wherein R¹ and R³ are as described above, with the corresponding sulphonylating agent (IX) in which X is an halogen atom such as chlorine, in the presence of a base such as triethylamine, diisopropylethylamine or pyridine and a catalytic amount of 4-dimethylaminopyridine in a solvent such as dichloromethane, pyridine, THF or acetonitrile at a temperature from 0°C to the boiling point of the solvent. Microwave may be used as an alternative heating procedure.

In the particular case in which L is a direct bond and n is 1, compounds of general formula lb may also be prepared as depicted in Figure 3.

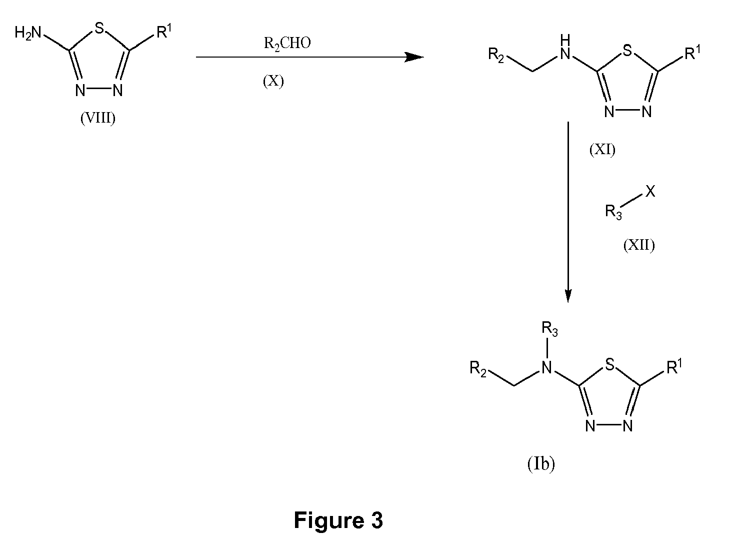

Compounds of general formula (Ib) may be prepared by the reaction of 1,3,4-thiadiazol-2-ylamine derivatives (XI), wherein R¹ and R² are as described above, with the corresponding alkylating agent (XII), wherein X is an halogen atom such as chlorine, bromine or iodide or a sulphonate such as mesylate, tosylate or triflate in basic media such as sodium hydride in a solvent such as THF or DMF at a temperature from 0 to 150°C. Microwave may be used as an alternative heating procedure. Intermediates of general formula (XI) may be prepared by reductive amination of compounds of general formula (VIII) with the corresponding aldehyde (X) following the same synthetic methods described above for preparing intermediates of formula (II) from intermediates of formula (VIII).

In the particular case where R¹ represents a group of formula 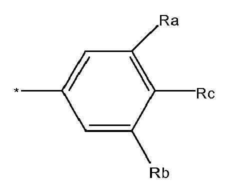 and Rc represents a C₁₋₄ alkoxy group of formula -OG, wherein G represents the alkyl radical of the alkoxy group which is substituted with one or more substituents, the compounds of formula (Ic) may be obtained following the synthetic path shown in Figure 4.

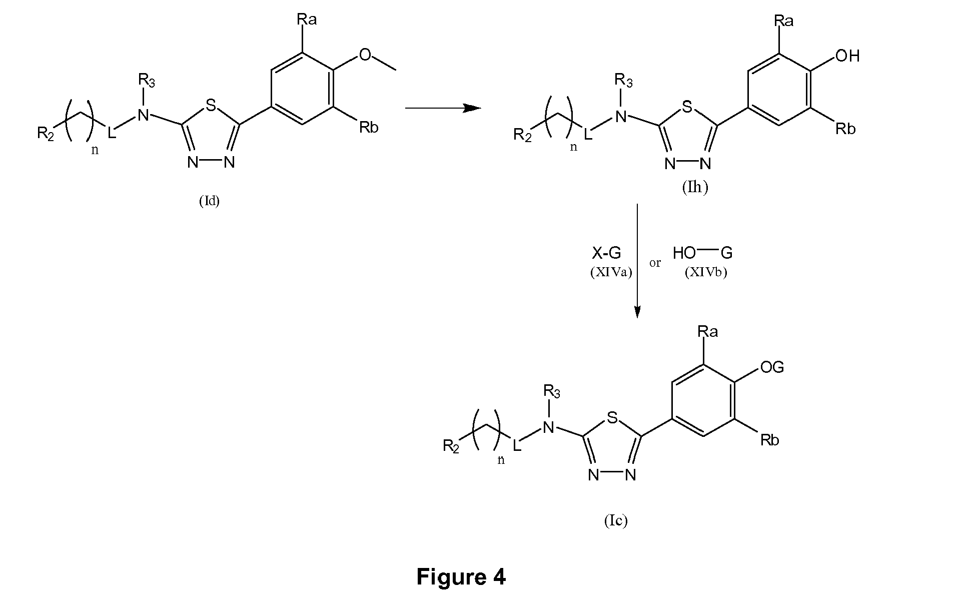

The compounds of formula (Ic) may be obtained by the reaction of 1,3,4-thiadiazol-2-aminophenol derivatives of formula (Ih), wherein Ra, Rb, R², R³ are as described above, with the corresponding alkylating agent (XIVa), wherein X is an halogen atom such as chlorine, bromine or iodide or a sulphonate such as mesylate, tosylate or triflate in basic media such as sodium hydride in a solvent such as THF or DMF at a temperature from 0 to 150°C. Alternatively, the phenolic functionality of (Ih) may be coupled to suitable alcohol derivatives (XIVb) HO-G, wherein G is as defined above, using a Mitsunobu coupling procedure (Mitsunobu, O., Synthesis 1 (1981)). Preferred coupling conditions include the use of a trialkylphosphine or triarylphosphine, such as tri-n-butylphosphine or triphenylphosphine, in a suitable solvent, such as tetrahydrofuran or dichloromethane, and an azodicarbonyl reagent, such as diethyl azodicarboxylate or 1,1'-(azodicarbonyl)dipiperidine.

The compounds of general formula (Ih) may be prepared by demethylation of the corresponding compound of general formula (Id) using BBr₃ or AIBr₃ or BF₃ or iodotrimethylsilane as demethylating agent in a solvent such as dichloromethane or 1,2-dichloroethane, chloroform at a temperature between 0 and the 60°C. Alternatively compounds of general formula (Ih) may be prepared by demethylation using HBr in acetic acid as a solvent.

In the particular case where R¹ represents a group of formula 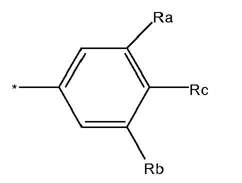 and Rc is a methyl group substituted with one or more -NR'R" substituents, wherein R' and R" are as defined above and L is a direct bond, the compounds of formula (Ie) may be obtained following the synthetic path shown in Figure 5.

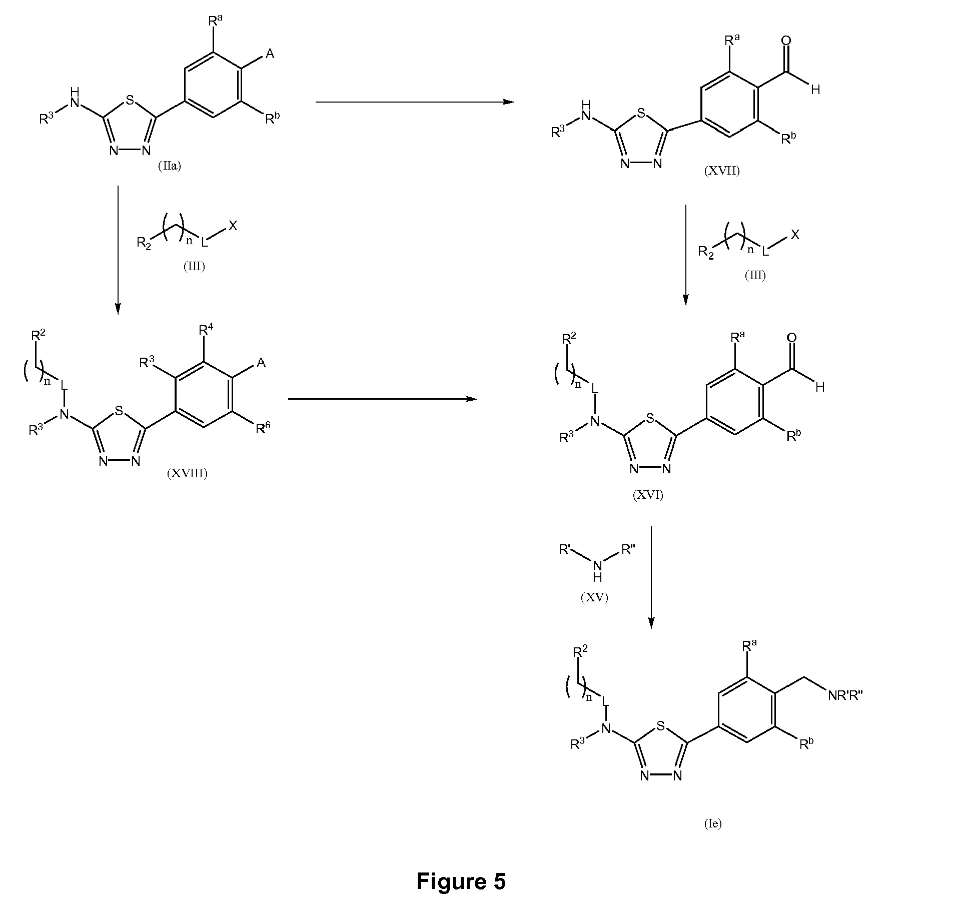

Compounds of general formula (Ie), wherein R², R³, R^{a}, R^{b} R' and R" are as described above may be prepared by the reductive amination of the aldehyde derivatives of general formula (XVI) with the corresponding amines of formula (XV) in acidic media such as acetic acid and in a protic solvent such as methanol or ethanol and with a reductive agent such as sodium borohydride, sodium triacetoxyborohydride or sodium cyanoborohydride at a temperature from 0°C to the boiling point of the solvent. As an alternative to the acidic media a Lewis acid such as zinc chloride can be used.

Compounds of formula (XVI) may be obtained by alkylation of compounds of general formula (XVII) with the corresponding alkylating agent of formula (III) in which X is as described above by standard methods as described before.

Finally, aldehyde derivatives of general formula (XVII) may be obtained by reduction of the corresponding nitrile of general formula (IIa), wherein A represents CN, by using a reductive agent such as diisobutylaluminium hydride (DIBAL-H) in a solvent such as dichloromethane, THF or dioxane at a low temperature from -78° to 0°C.

Alternatively, compounds of general formula (XVII) may also be obtained by reduction of the corresponding ester (IIa), wherein A represents a -COOR radical, to the alcohol using LiAlH₄ as reductive agent in a solvent such as THF followed by oxidation to the corresponding aldehyde of formula (XVII) by a standard Swern oxidation or other oxidazing agents such as Dess-Martin reagent.

Alternatively, compounds of general formula (XVII) may also be obtained by dihydroxylation and oxidative cleavage of the corresponding vinyl derivative (IIa), weherein A represents a -CH=CH₂ group, using a catalytic amount of an oxidazing agent such as osmium tetroxide and a cooxidant such as N-methylmorpholine-N-oxide followed by the addition of sodium periodate in a mixture of solvents such as methanol, acetonitrile, acetone and water at a temperature from 0°C to the boiling point of the mixture. This cleavage may also be performed by ozonolysis. Thus, ozone is bubbled through a solution of compounds of general formula (IIa) wherein A represents a - CH=CH₂ radical in a solvent such as dichloromethane at -78 °C followed by the addition of a reductive agent such as triphenylfosfine, thiourea, zinc dust or dimethylsulfide. A cosolvent such as methanol may be then added to the reaction mixture and the reaction is performed at room temperature.

Alternatively, compounds of formula (XVI) may be obtained from compounds of formula (XVIII) according to the methods described above, depending on the nature of A. Compounds of formula (XVIII) can be obtained from compounds of formula (IIa) following the same procedures described for the obtention of intermediates of formula (XVI) from intermediates of formula (XVII).

In the particular case where R¹ represents a group of formula 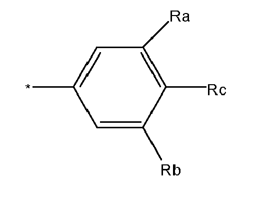 and Rc is a methyl group substituted with one -NR'R" substituents, wherein R' and R" are as defined above and L is a -S(O)₂ group, the compounds of formula (If) may be obtained following the synthetic path shown in Figure 6.

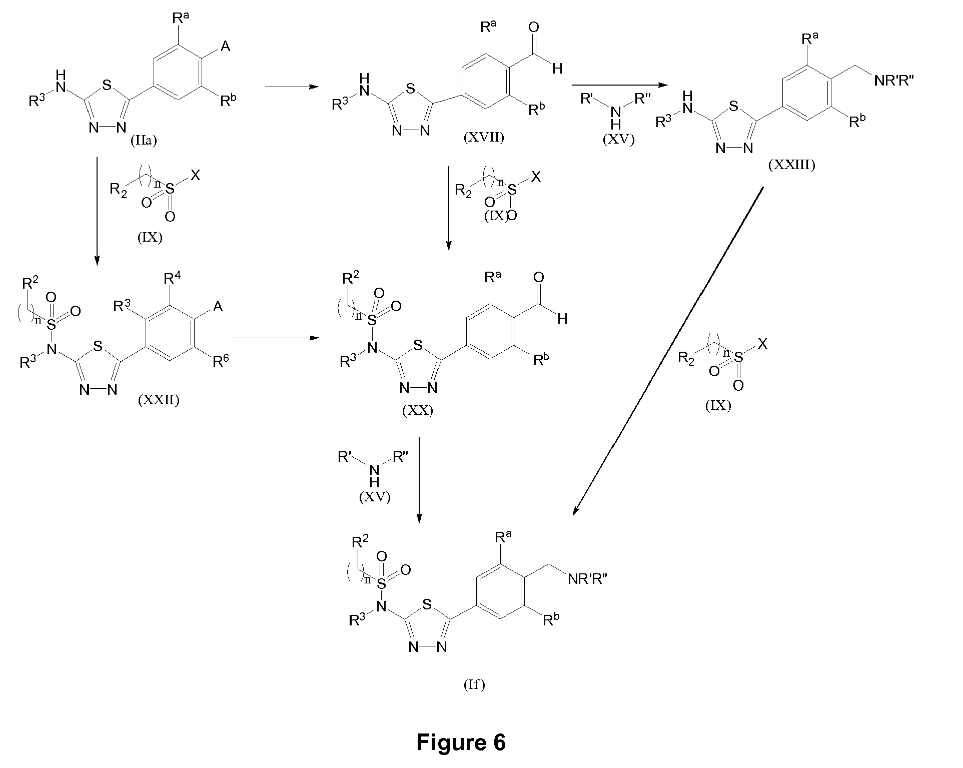

Compounds of general formula (If), wherein R², R³, R^{a} and R^{b} are as described above may be prepared by the reductive amination of the aldehyde derivatives of general formula (XX) with the corresponding amines of formula (XV) in acidic media such as acetic acid and in a protic solvent such as methanol or ethanol and with a reductive agent such as sodium borohydride, sodium triacetoxyborohydride or sodium cyanoborohydride at a temperature from 0°C to the boiling point of the solvent. As an alternative to the acidic media a Lewis acid such as zinc chloride can be used.

Compounds of formula (XX) may be obtained by sulphonylation of compounds of general formula (XVII) with the corresponding sulphonylating agent of formula (IX) in which X is as described above by standard methods as described before.

Alternatively, compounds of formula (XX) may be obtained from compounds of formula (XXII) following the synthetic methods described in Figure 5, depending on the nature of A. Compounds of formula (XXII) can be obtained from compounds of formula (IIa) following the same procedures described for the obtention of compounds of formula (XX).

Alternatively, compounds of general formula (If) can be obtained by sulphonylation of compounds of general formula (XXIII) with the corresponding sulphonylating agent of formula (IX) in which X is as described above by standard methods as described before. Compounds of general formula (XXIII) can be in turn obtained by reductive amination of aldehydes of general formula (XVII) with amines of general formula (XV) by the methods described above.

In the particular case where R¹ represents a group of formula 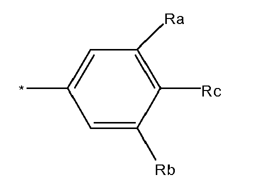 and Rc is an ethoxy group substituted with one or more -NR'R" substituents, wherein R' and R" are as defined above and L is direct bond, the compounds of formula (Ig) may be obtained following the synthetic path shown in Figure 7.

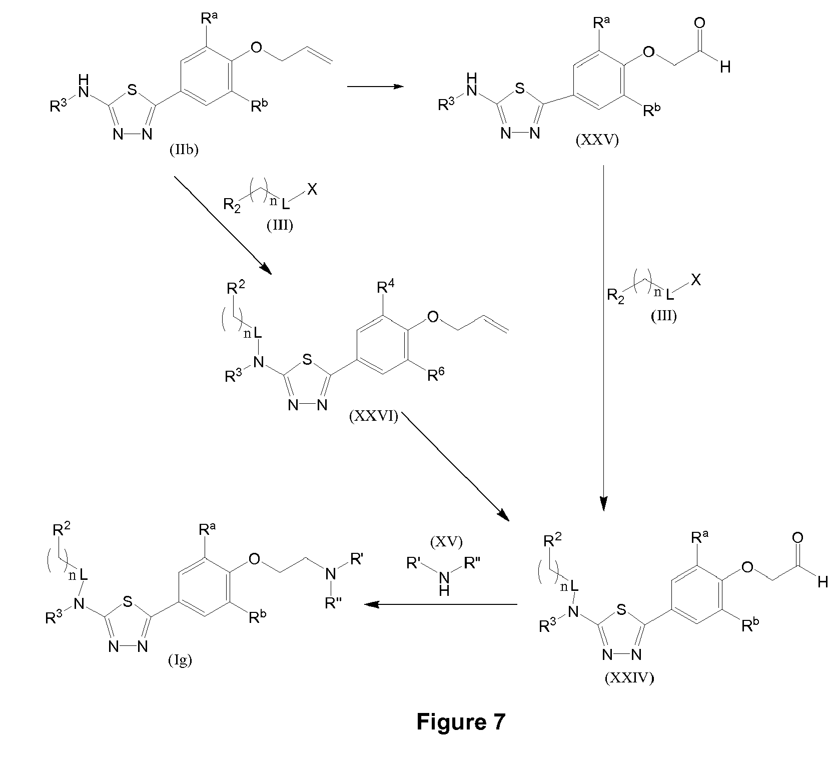

Compounds of general formula (Ig), wherein R², R³, R^{a} and R^{b} are as described above may be prepared by the reductive amination of the aldehyde derivatives of general formula (XXIV) with the corresponding amines of formula (XV) by the methods described above.

Compounds of formula (XXIV) may be obtained by alkylation of compounds of general formula (XXV) with the corresponding alkylating agent of formula (III) in wherein L is a direct bond, R² is as described above and X is an halogen atom such as chlorine, bromine or iodine or a sulphonate such as mesylate, tosylate or triflate by standard methods as described before.

Compounds of general formula (XXV) may be obtained by dihydroxylation and oxidative cleavage of the corresponding allyl derivative (IIb) using a catalytic amount of an oxidazing agent such as osmium tetroxide and a cooxidant such as sodium periodate in a mixture of solvents such as methanol, acetonitrile, acetone and water at a temperature from 0°C to the boiling point of the mixture. This cleavage may also be performed by ozonolysis. Thus, ozone is bubbled through a solution of compounds of general formula (IIb) in a solvent such as dichloromethane at -78 °C followed by the addition of a reductive agent such as triphenylfosfine, thiourea, zinc dust or dimethylsulfide. A cosolvent such as methanol may be then added to the reaction mixture and the reaction is performed at room temperature.

Alternatively, compounds of formula (XXIV) may be obtained from compounds of formula (XXVI) by oxidative cleavage, according to the method described above. Compounds of formula (XXVI) can be obtained from compounds of formula (IIb) by alkylation with compounds of general formula (III) following the same procedures described for the obtention of compounds of formula (XXIV) from intermediates of formula (XXV).

When the defined groups R¹ to R³ and Ra to Rb are susceptible to chemical reaction under the conditions of the hereinbefore described processes or are incompatible with said processes, conventional protecting groups may be used in accordance with standard practice, for example see T.W. Greene and P.G.M. Wuts in "protective Groups in Organic Chemistry", 3rd Edition, John Wiley&Sons (1999). It may be that deprotection will form the last step in the synthesis of compounds of formula (I).

The syntheses of the compounds of the invention and their intermediates are illustrated by the following Examples (1 to 45) including Preparation Examples (1 to 40) which do not limit the scope of the invention in any way
Starting compounds are commercially available or may be obtained following the conventional synthetic method already known in the art.

¹H Nuclear Magnetic Resonance Spectra were recorded on a Varian Mercury 200 spectrometer. Low Resolution Mass Spectra (m/z) were recorded on a Micromass ZMD mass spectrometer using ESI ionization. The chromatographic separations were obtained using a Waters 2690 system equipped with a Symmetry C18 (2.1 x 50 mm, 3.5 µM) column for method A and B and a Symmetry C18 (2.1 x 100 mm, 3.5 µM) for method C. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A), the gradients are specified in the following table for each methode used. The reequilibration time between two injections was 1 min. The flow rate was 0.8 mL/min for method A and 0.4 mL/min for method B and C. The injection volume was 5 microliter for method A and B and 3 microliter for method C. Diode array chromatograms were collected at 210 nM.

| Method | 0% B | 0 to 95% B | 95% B |
|---|---|---|---|
| A | 0.2 min | 3min | 0.8min |
| B | 0.5min | 6.5min | 1min |
| C | 0min | 20min | 4min |

### PREPARATIONS and EXAMPLES

### PREPARATION 1

### N-butylhydrazinecarbothioamide

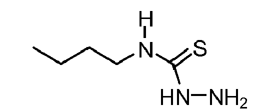

To a 10°C stirred solution of hydrazine hydrate (32 ml, 0.66 mol) in methanol (500 ml), butylisothiocyanate (25 g, 0.22 mol) was added dropwise keeping the temperature between 10-15°C. The final mixture was stirred for 1 h at 10°C and the solvent was removed to yield an oil that was lyophilized. A white solid was obtained. It was washed with diethyl ether to yield 28g (87% yield).
LRMS: m/z 148 (M+1)⁺
Retention time: 4.17min (method B)
¹H NMR (200 MHz, CDCl₃) δ ppm 1.0 (t, *J*=7.0 Hz, 3 H) 1.4 (m, 2 H) 1.6 (m, 2 H) 3.6 (m, 2 H) 3.8 (s, 2 H) 7.4 (s, 1 H) 7.8 (s, 1 H)

### PREPARATION 2

### N-butyl-5-(4-methoxy-3,5-dimethylphenyl)-1,3,4-thiadiazol-2-amine

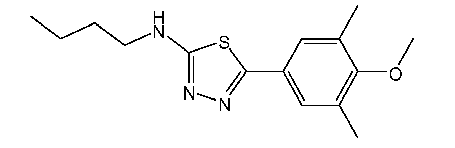

To a stirred suspension of 4-methoxy-3,5-dimethylbenzoic acid (4.40 g, 24.4 mmol) and N-butylhydrazinecarbothioamide (Preparation1) (3 g, 20.4 mmol) in dioxane (30 ml), a solution of POCl₃ (3.70 ml, 40.5 mmol) in dioxane (5ml) was added dropwise at room temperature and the final mixture was stirred at 80°C for 5 h. The mixture was left to cool down. Water (5ml) was added dropwise to the stirring mixture at 0-5°C and it was carefully poured onto ice water. To the suspension obtained sodium hydroxide (32%) was added dropwise to reach pH 9-10. It was stirred for 30 min. The solid thus formed was filtered, thoroughly washed with water and suspended in isopropanol (25ml). It was stirred and cooled down to 0-5°C. The solid obtained was filtered, washed with isopropyl ether and dried to yield 5.22 g (88%) of the title compound.
LRMS: m/z 292(M+1)⁺
Retention time: 6.67min (method B)
¹H NMR (200 MHz, DMSO-*d*₆) δ ppm 0.92 (t, *J*=7.22 Hz, 3 H) 1.39 (dq, *J*=14.54, 7.13 Hz, 2 H) 2.28 (m, 2 H) 3.38 (t, *J*=6.64 Hz, 2 H) 3.70 (s, 3 H) 4.58 - 5.60 (m, 6 H) 7.48 (s, 2 H).

### PREPARATION 3

### 5-(4-methoxy-3,5-dimethylphenyl)-1,3,4-thiadiazol-2-amine

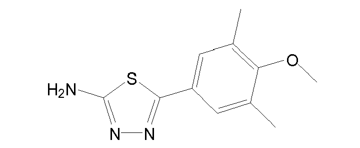

To a stirred suspension of 4-methoxy-3,5-dimethylbenzoic acid (5 g, 27.8 mmol) and hydrazinecarbothioamide (2.78 g, 30.5 mmol) in dioxane (80 ml), a solution of POCl₃ (5.10 ml, 55.9 mmol) in dioxane (5ml) was added dropwise at room temperature and the final mixture was stirred at 80°C for 5 h. The mixture was left to cool down and it was carefully poured onto ice water. Dichloromethane (1 L) and a 4% aqueous solution of sodium hydrogencarbonate (to reach pH 8-9) were added to the mixture obtained. It was stirred and the two layers were left to separate overnight. The organic phase was dried, filtered and concentrated in vacuo. The solid obtained was treated with isopropanol, filtered, washed with hexane and dried to yield 2.68 g (41%) of the title compound.
LRMS: m/z 236 (M+1)⁺
Retention time: 5.22 min (method B)
*¹H NMR (200 MHz, DMSO-d₆)* δ *ppm* 2.25 *(s,* 6 *H) 3.70 (s, 3 H) 7.30 (s, 2H) 7.50 (s, 2 H)*

### PREPARATION 4

### N-(2-chlorobenzyl)-5-(4-methoxy-3,5-dimethylphenyl)-1,3,4-thiadiazol-2-amine

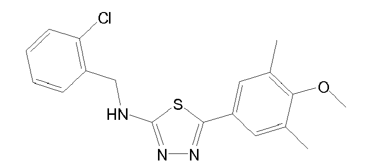

To a stirred suspension of the title compound of Preparation 3 (2.76 g, 11.7 mmol) in ethanol (30 ml), 2-chlorobenzaldehyde (4 ml, 35.5 mmol) and acetic acid (0.34 ml, 5.9 mmol) were addded. It was stirred at 70°C for 48h. It was left to cool down to room temperature and sodium borohydride (1.33 g, 35.2 mmol) was carefully added. It was stirred for 2h. The mixture was concentrated in vacuo and water and ethyl acetate were added to the residue obtained. The organic phase was washed with water and brine, dried and concentrated in vacuo. The solid obtained was treated with ethyl ether, filtered and dried to yield 1.83 g (41 %) of the title compound.
LRMS: m/z 360 (M+1)⁺
Retention time: 6.93 min (method B)
¹H NMR (200 MHz, CHLOROFORM-d) δ ppm 2.30 (s, 6 H) 3.70 (s, 3 H) 4.70 (s, 2H) 5.75 (s, 1 H) 7.20 - 7.25 (m, 2 H) 7.30 - 7.60 (m, 4 H)

### PREPARATION 5

### N-butyl-5-(2-methylpyridin-4-yl)-1,3,4-thiadiazol-2-amine

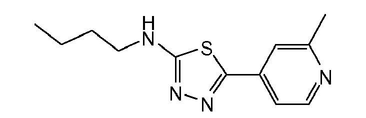

To a stirred suspension of 2-methylisonicotinic acid (a triethylamine salt form) (0.68 g, 4.96 mmol) a 2N aqueous solution of HCl was added to reach acid pH 1-2. The solution obtained was extracted with ethylacetate. The expected compound was not detected in the organic phase. The aqueous phase was concentrated in vacuo. The residue obtained was suspended in ethanol. It was concentrated in vacuo to yield 0.63 g of the acid form of the 2-methylisonicotinic acid.

To a stirred suspension of 2-methylisonicotinic acid (0.63 g, 4.9 mmol) in dioxane (10 ml) the title compound of Preparation 1 (0.73 g g, 4.9 mmol) was added. To the suspension thus obtained a solution of POCl₃ (0.90 ml, 9.9 mmol) in dioxane (3 ml) was added dropwise. The mixture was stirred at 80°C for 3.5h. The upper layer of the mixture obtained was poured and water was added to the lower layer. To the aqueos solution thus obtained a solution of potassium carbonate was added to reach pH 7-8 and it was extracted with dichloromethane. The organic phase was washed with brine, dried and concentrated in vacuo. The residue obtained was treated with isopropyl ether, filtered, and dried to yield 0.61g (50%) of the title compound.
LRMS: m/z 249 (M+1)⁺
Retention time: 4.39 min (method B)
1 H NMR (200 MHz, CHLOROFORM-*d*) δ ppm 0.96 (q, *J*=6.77 Hz, 3 H) 1.32 - 1.56 (m, 2 H) 1.62 - 1.86 (m, 2 H) 2.62 (s, 3 H) 3.40 (t, *J*=7.03 Hz, 2 H) 6.18 (s, 1 H) 7.48 (d, *J*=5.08 Hz, 1 H) 7.49 - 7.59 (m, 1 H) 8.55 (d, *J*=5.08 Hz, 1 H)

### PREPARATION 6

### N-butyl-5-imidazo[1,2-a]pyridin-6-yl-1,3,4-thiadiazol-2-amine

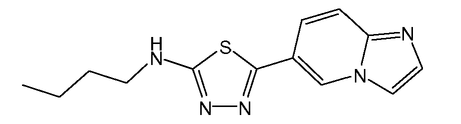

To a stirred suspension of imidazo[1,2-a]pyridine-6-carboxylic acid (1 g, 6.2 mmol) in dioxane (12 ml) the title compound of Preparation 1 (0.91 g, 6.2 mmol) was added. To the suspension thus obtained POCl3 (1.73 ml, 18.6 mmol) was added dropwise at room temperature. The mixture was stirred at 70°C for 3h under inert atmosphere. It was poured onto a mixture of a 2M aqueous solution of sodium hydroxide (50 ml) and ice (50 ml). The solid obtained was filtered, washed and dried to yield 792 mg (47%) of the title compound.
LRMS: m/z 274 (M+1)⁺
Retention time: 2.08min (method A)
¹H NMR (200 MHz, DMSO-d₆) δ ppm 0.98(t, J=8 Hz, 3H), 1.46(m, 2H), 1.69(m, 2H), 3.41 (t, J=8Hz, 2H), 5.48 (brs, 1 H), 7.65(m, 4H), 8.64(s, 1 H)

### PREPARATION 7

### N-butyl-5-(4-vinylphenyl)-1,3,4-thiadiazol-2-amine

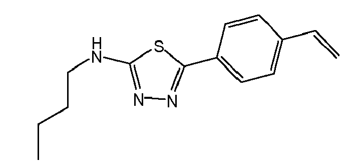

To a stirred solution of 4-vinylbenzoic acid (5.40 g, 36.45 mmol) in dioxane (70 ml) POCl₃ was added dropwise (8.50 g, 55.44 mmol). It was stirred at 90°C for 1 h. It was allowed to cool down to room temperature and the title compound of Preparation 1 was added ((5.90 g, 40.07 mmol). It was stirred at room temperature overnight and then it was heated at 100°C for 4h. The reaction mixture was poured onto water/NaOH (2M) and the solid obtained was filtered. It was dissolved in dichloromethane and washed with water. The organic phase was dried, filtered and concentrated in vacuo to give 3.68 g (34%) of the title compound.
LRMS: m/z 260 (M+1)⁺
Retention time: 6.75 min (method B)
¹H NMR (200 MHz, DMSO-*d*₆) δ ppm 0.91 (t, *J*=7.22 Hz, 3 H) 1.23 - 1.49 (m, 2 H) 1.48 - 1.72 (m, 2 H) 3.21 - 3.40 (m, 2 H) 5.34 (d, *J*=10.93 Hz, 1 H) 5.92 (d, *J*=17.57 Hz, 1 H) 6.78 (dd, *J*=17.57, 10.93 Hz, 1 H) 7.45 - 7.87 (m, 4 H) 7.99 (t, 1 H).

### PREPARATION 8

### N-butyl-5-(2-chloro-6-methoxypyridin-4-yl)-1,3,4-thiadiazol-2-amine

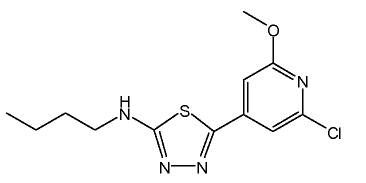

To a stirred suspension of 2-chloro-6-methoxyisonicotinic acid (0.94 g, 5.0 mmol) in dioxane (10 ml) the title compound of Preparation 1 (0.74 g, 5.0 mmol) was added. To the suspension thus obtained POCl₃ (1.40 ml, 15.1 mmol) was added dropwise at room temperature. The mixture was stirred at 70°C for16h. It was poured onto a mixture of a 2M aqueous solution of sodium hydroxide (50 ml) and ice (50 ml). The solid obtained was filtered, washed with water and hexane and dried to yield 1.23 g (83%) of the title compound.
LRMS: m/z 299 (M+1)⁺
Retention time: 3.61 min (method A) ¹H NMR (200 MHz, DMSO-D6) δ ppm 0.9 (t, *J*=7.2 Hz, 3 H) 1.4 (m, 2 H) 1.6 (m, 2 H) 3.3 (m, 2 H) 3.9 (s, 3 H) 7.1 (d, *J*=1.2 Hz, 1 H) 7.4 (d, *J*=1.2 Hz, 1 H) 8.4 (t, *J*=6.1 Hz, 1 H)

### PREPARATION 9

### 4-[5-(butylamino)-1,3,4-thiadiazol-2-yl]-2,6-dimethylphenol

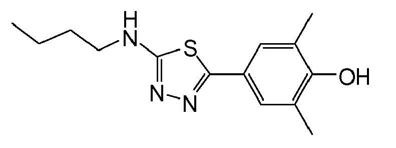

To a stirred suspension of the title compound of Preparation 2 (2 g, 6.86 mmol) in dry dichloromethane (20 ml) cooled down to -78°C a 1 M solution of BBr₃ in dichloromethane (10.3 ml, 10.3 mmol) was added dropwise. It was stirred for 30 min under the previous conditions. It was left to warm up to room temperature and it was stirred for 12h. Water/ice were added and the mixture was diluted with more dichloromethane (to reach 200 ml). A 4% aqueous sodium hydrogen carbonate solution was added to reach pH 7-8. The organic phase was concentrated in vacuo and the residue obtained was treated with diethyl ether. It was filtered and dried to yield 1.90 g (87%) of the title compound.
LRMS: m/z 278 (M+1)⁺
Retention time: 6.09min (method B-TDA1)
¹H NMR (200 MHz, DMSO-D6) δ ppm 0.9 (t, *J*=7.2 Hz, 3 H) 1.4 (m, 2 H) 1.6 (m, 2 H) 2.2 (s, 6 H) 3.3 (m, 2 H) 7.3 (s, 2 H) 8.0 (s, 1 H) 8.8 (s, 1 H)

### PREPARATION 10

### N-butyl-5-(6-methoxypyridin-3-yl)-1,3,4-thiadiazol-2-amine

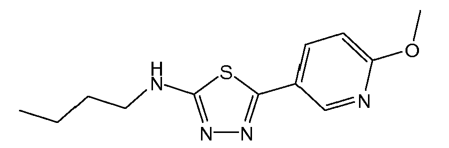

To a stirred suspension of 6-methoxynicotinic acid (2.26 g, 14.8 mmol) in dioxane (38 ml) the title compound of Preparation 1 (2.17 g, 14.7 mmol) was added. To the suspension thus obtained POCl3 (4.10 ml, 44.1 mmol) was added dropwise at room temperature. The mixture was stirred at 70°C for 2h under inert atmosphere. It was poured onto a mixture of a 2M aqueous solution of sodium hydroxide (100 ml) cooled down to 0-5°C. The mixture obtained was extracted with dichloromethane. The organic phase was washed with water, dried and concentrated in vacuo. The residue obtained was purified by reverse chromatography to yield 0.58 g (15%) of the title compound.
LRMS: m/z 265 (M+1)⁺
Retention time: 3.06min (method A)
¹H NMR (200 MHz, DMSO-d₆) δ ppm 0.91(t, J=8 Hz, 3H), 1.39(m, 2H), 1.58(m, 2H), 3.31 (q, J=6Hz, 2H), 3.90(s,3H), 6.93(d,J=8Hz, 1 H), 7.94 (m,1H), 8.09 (d,J=8Hz, 1 H), 8.52 (s, 1 H)

### PREPARATION 11

### imidazo[1,2-a]pyridine-7-carboxylic acid

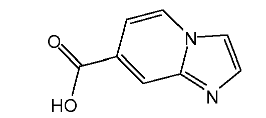

A stirred solution of methyl imidazo[1,2-a]pyridine-7-carboxylate (2.12 g, 12.0 mmol) in a 37% HCl solution (100 ml) was heated at 50°C for 64h. The mixture was concentrated in vacuo and dried at 40°C in vacuo to yield 2.3 g (100%) of the HCl salt of the title compound
LRMS: m/z 163 (M+1)⁺
Retention time: 0.41 min (method A)
¹H NMR (200 MHz, DMSO-d₆) δ ppm 7.79 (d, J=8Hz, 1 H), 8.31 (s, 1 H), 8.38(s,1H), 8.48(s,1H), 8.97(d,J=8Hz, 1 H)

### PREPARATION 12

### N-butyl-5-imidazo[1,2-a]pyridin-7-yl-1,3,4-thiadiazol-2-amine

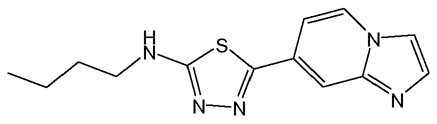

To a stirred suspension of imidazo[1,2-a]pyridine-7-carboxylic acid (preparation 11) (1.66 g, 10.2 mmol) in dioxane (30 ml) the title compound of Preparation 1 (1.68 g, 11.4 mmol) was added. To the suspension thus obtained POCl3 (3.18 ml, 34.2 mmol) was added dropwise at room temperature. The mixture was stirred at 70°C for 3h under inert atmosphere. It was concentrated in vacuo and then re-suspended in the same solvent (5ml). The mixture was poured onto a mixture of a 2M aqueous solution of sodium hydroxide (50 ml) and ice (50 ml). The solid obtained was filtered, washed with water and dried to yield 2.13 g (76%) of the title compound.
LRMS: m/z 274 (M+1)⁺
Retention time: 1.97min (method A)
¹H NMR (200 MHz, DMSO-D6) δ ppm 0.9 (t, *J*=6.2 Hz, 3 H) 1.4 (m, 2 H) 1.6 (m, 2 H) 3.3 (m, 2 H) 7.4 (d, *J*=6.6 Hz, 1 H) 7.7 (s, 1 H) 7.8 (s, 1 H) 8.1 (m, 2 H) 8.6 (d, *J*=5.1 Hz, 1 H)

### PREPARATION 13

### 4-(cyanomethyl)benzoic acid

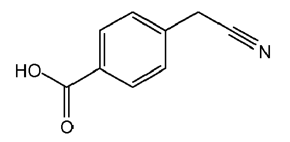

To a stirred solution of methyl 4-(cyanomethyl)benzoate (10 g, 60 mmol) in THF (500 ml) a solution of LiOH (2.90 g, 70 mmol) in water (600 ml) was added. It was stirred at room temperature for 16h. The mixture was concentrated in vacuo and onto the aqueous phase left a solution of HCl 35% (6ml) was added. The solid obtained was filtered, washed with water and hexane and dried in vacuo at 40°C for 64h. It was treated with hexane (100 ml) stirring for 1 h. The solid obtained was filtered and dried to yield 7.95 g (86%) of the title compound.
LRMS: m/z 260 (M-1)⁻
Retention time: 2.10 min (method A)

### PREPARATION 14

### {4-[5-(butylamino)-1,3,4-thiadiazol-2-yl]phenyl}acetonitrile

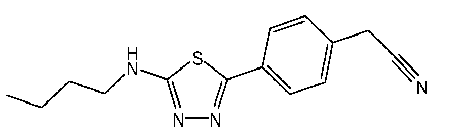

To a stirred solution of 4-(cyanomethyl)benzoic acid (Preparation 13) (1 g, 6.21 mmol) in dioxane (50 ml) the title compound of Preparation 1 (0.92 g, 6.21 mmol) was added. To the solution obtained a solution of POCl3 (1.16 ml, 12.43 mmol) in dioxane (10 ml) was added dropwise at room temperature. The mixture was stirred at 70°C for 16h. It was concentrated in vacuo and then re-disolved in dioxane (10 ml). The mixture was poured onto a 2M aqueous solution of sodium hydroxide (40 ml). The solid obtained was filtered, washed with water and hexane. It was dried to yield 0.91 g (54%) of the title compound.
LRMS: m/z 273 (M+1)⁺
Retention time: 2.98 min (method A) ¹H NMR (200 MHz, CHLOROFORM-*d*) δ ppm 0.98 (t, *J*=7.22 Hz, 3 H) 1.30 - 1.52 (m, 2 H) 1.52 - 1.79 (m, 2 H) 3.39 (t, *J*=6.83 Hz, 2 H) 3.80 (s, 2 H) 5.38 (s, 1 H) 7.41 (d, *J*=8.59 Hz, 2 H) 7.82 (d, *J*=8.59 Hz, 2 H)

### PREPARATION 15

### N-butyl-5-(3-chloro-4-methoxyphenyl)-1,3,4-thiadiazol-2-amine

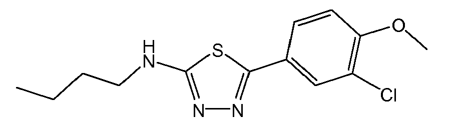

To a stirred solution of 3-chloro-4-methoxybenzoic acid (0.74 g, 3.99 mmol) in dioxane (5 ml) the title compound of Preparation 1 (0.59 g, 3.99 mmol) was added. To the solution thus obtained POCl₃ (1.12 ml, 11.96 mmol) was added dropwise at room temperature. The suspension was stirred at 70°C for 16h. The mixture was poured onto water (100 ml) at 0-5°C and the solid obtained was filtered and washed with water and hexane. It was treated with isopropanol (10 ml) at 70°C. To the solution obtained a 2M aqueous solution of NaOH (50 ml) was added. The solid thus obtained was filtered and washed with hexane. It was adried in vacuo for 48h to yield 0.82g (69%) of the title compound.
LRMS: m/z 298 (M+1)⁺
Retention time: 3.44min (method A)
¹H NMR (200 MHz, DMSO-D6) δ ppm 0.9 (t, *J*=7.2 Hz, 3 H) 1.4 (m, 2 H) 1.6 (m, 2 H) 3.3 (m, 2 H) 3.9 (s, 3 H) 7.2 (d, *J*=9.0 Hz, 1 H) 7.7 (dd, *J*=8.6, 2.3 Hz, 1 H) 7.8 (d, *J*=2.3 Hz, 1 H) 7.9 (t, *J*=5.5 Hz, 1 H)

### PREPARATION 16

### 4-(Allyloxy)-3,5-dimethylbenzonitrile

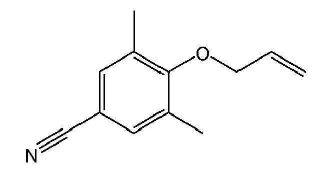

To a stirred solution of 4-hydroxy-3,5-dimethylbenzonitrile (10 g, 68 mmol) in DMF (100 ml) cooled down to 0-5°C sodium hydride (3 g, 75 mmol) was added. It was stirred under the previous conditions for 20 min. A solution of 3-bromoprop-1-ene (6.04 ml, 71.39 mmol) in DMF (25 ml) was added dropwise. The mixture obtained was left to warm up to room temperature and it was stirred overnight under inert atmosphere. Sodium hydride (1.50 g, 37.5 mmol) and 3-bromoprop-1-ene (3.02, 35.7 mmol) were added to the mixture and it was stirred at room temperature for 2h more under inert atmosphere.

It was carefully pouredonto water/ice (1 I) and the suspension obtained was filtered. The solid was washed with water and hexane. It was dried to yield 14.45 g (113%) of the title compound slightly impure (traces of parafine/ mineral oil coming from the NaH used were detected by ¹H-NMR).
LRMS: m/z 188 (M+1)⁺
Retention time: 6.52 min (method B)
1 H NMR (400 MHz, CHLOROFORM-*d*) δ ppm 2.29 (s, 6 H) 4.34 (d, *J*=5.86 Hz, 2 H) 5.29 (dd, J = 10.4, 1.1 Hz, 1 H) 5.43 (dd, *J*=17.19, 1.56 Hz, 1 H) 6.02 - 6.15 (m, 1 H), 7.33 (s, 2 H)

### PREPARATION 17

### 4-(Allyloxy)-3,5-dimethylbenzoic acid

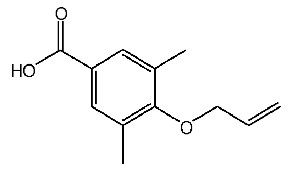

To a stirred solution of the title compound of preparation 16 (12.45 g, 66.5 mmol) in ethanol (12 ml) an aqueous 8N solution of sodium hydroxide (65 ml) was added. It was stirred at 110 °C for 7h in a pressure reactor. Water (100 ml) and dichloromethane (200 ml) were added to the mixture. It was cooled down to 0-5 °C and a 6N aqueous solution of HCl was added to reach pH1. More water and dichloromethane were added and the two phases were separated. The aqueous phase was extracted with dichloromethane. The organic phases were collected together, washed with water and brine, dried and concentrated in vacuo.The residue obtained was treated with hexane, filtered and dried to yield 12.23 g (89%) of the title compound.
LRMS: m/z 207 (M+1)⁺
Retention time: 3.15 min (method A)

### PREPARATION 18

### 5-[4-(allyloxy)-3,5-dimethylphenyl]-N-butyl-1,3,4-thiadiazol-2-amine

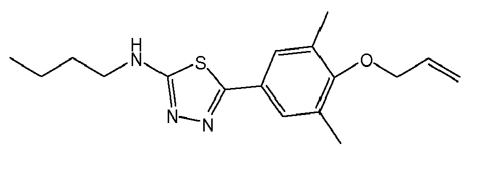

To a stirred suspension of 4-(allyloxy)-3,5-dimethylbenzoic acid ( 10.43 g, 50.57 mmol) in dioxane (100 ml) the title compound of Preparation 1 (8.10 g, 55.01 mmol) was added. To the solution thus obtained POCl₃ (8 ml, 87.65 mmol) was added dropwise at room temperature. The suspension was stirred at 80°C for 5h. The mixture was concentrated in vacuo and the residue obtained was suspended in water and dichloromethane. An aqueous solution 2N of sodium hydroxide was added to reach pH 8-9 and a solid precipitated. Dichloromethane was added to disolve the solid (500 ml approx.) and the organic phase was washed with brine and dried. It was concentrated in vacuo and the residue obtained was treated with a mixture of diethyl ether/hexane. It was filtered, washed and dried to yield 11.72 g (73%) of the title compound.
LRMS: m/z 318 (M+1)⁺
Retention time: 3.68 min (method A)

### PREPARATION 19

### 5-[4-(Allyloxy)-3,5-dimethylphenyl]-N-butyl-N-(2-fluorobenzyl)-1,3,4-thiadiazol-2-amine

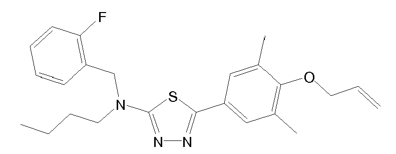

To a stirred solution of 5-(4-(allyloxy)-3,5-dimethylphenyl)-N-butyl-1,3,4-thiadiazol-2-amine (Preparation 18) (2 g, 6.30 mmol) in THF (20 ml) sodium hydride (0.6 g, 15 mmol) was added. It was stirred at room temperature for 30 min. and cooled down to 0-5 °C. A solution of 1-(bromomethyl)-2-fluorobenzene (1.2 ml, 9.95 mmol) in THF (25 ml) was added dropwise. The mixture was allowed to warm up to room temperature and it was stirred under these conditions for 35 min under inert atmosphere. It was heated at 60 °C for 1h 15 min. The mixture was poured onto water/ice (300 ml) and an aqueous 2N solution of HCl (10 ml). It was extracted with ethyl acetate and the organic phase was dried and concentrated in vacuo. The residue obtained was purified by chromatography (SiO2, Hexane; Hexane/ethyl acetate 9:1) to yield 2.48 g (93%) of the title compound.
LRMS: m/z 426 (M+1)⁺
Retention time: 7.93 (method B)
¹H NMR (200 MHz, DMSO-*d*₆) δ ppm 0.89 (t, *J*=7.22 Hz, 3 H) 1.25 - 1.43 (m, 2) 1.47 - 1.71 (m, 2 H) 2.25 (s, 6 H) 3.40 - 3.58 (m, 2 H) 4.34 (d, *J*=5.47 Hz, 2 H), 4.79 (s, 2 H) 5.26 (d, *J*=10.15 Hz, 1 H) 5.34 - 5.60 (m, 1 H) 5.90 - 6.27 (m, 1 H), 96 - 7.70 (m, 6 H)

### PREPARATION 20

### (4-{5-[Butyl(2-fluorobenzyl)amino]-1,3,4-thiadiazol-2-yl}-2,6-dimethylphenoxy)-acetaldehyde

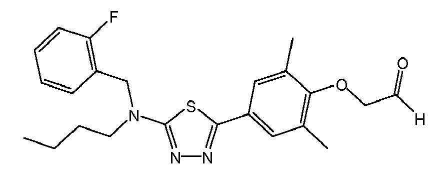

To a stirred solution of 5-(4-(allyloxy)-3,5-dimethylphenyl)-N-butyl-N-(2-fluorobenzyl)-1,3,4-thiadiazol-2-amine (Preparation 19) (2.45 g, 5.76 mmol) in THF (50 ml) 4-methylmorpholine N-oxide (1.89 g, 16.13 mmol) and a 4% aqueous solution of osmium tetroxide (1.06 ml, 0.17 mmol) were added. It was stirred at room temperature overnight under inert atmosphere.The mixture was concentrated in vacuo and water and ethyl acetate were added to the residue obtained. The aqueous phase was extracted with more ethyl acetate and the organic phases were collected together, washed with water and brine, dried and concentrated in vacuo. The residue obtained (3.42 g) was disolved in methanol (65 ml) and water (7 ml). To this solution sodium periodate (2.75 g, 12.86 mmol) was added and it was stirred at room temperature overnight. The mixture was concentrated in vacuo and water and ethyl acetate were added to the residue obtained. The aqueous phase was extracted with more ethyl acetate and the organic phases were collected together, washed with water and brine, dried and concentrated in vacuo to yield 2.5 g (100%) of the title compound.
LRMS: m/z 460 (M+MeOH)⁺
Retention time: 7.35 min (method B)
¹H NMR (200 MHz, DMSO-*d*₆) δ ppm 0.89 (t, *J*=7.22 Hz, 3 H) 1.19 - 1.42 (m, 2 H) 1.46 -1.74 (m, 2 H) 2.26 (s, 6 H) 3.41 - 3.55 (m, 2 H) 3.59 - 3.85 (m, 2 H) 4.70 - 4.88 (m, 2 H) 7.04 - 7.28 (m, *J*=7.42 Hz, 2 H) 7.31 - 7.52 (m, 4 H) 9.72 (s, 1 H)

### PREPARATION 21

### N-Cyclopentylhydrazinecarbothioamide

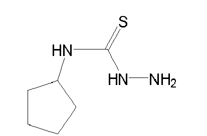

To a solution of isothiocyanatocyclopentane (2 g, 15.72 mmol) in ethanol (17 ml) hydrazine hydrate (0.7 ml, 14.36 mol) was added. It was stirred at room temperature for 1.5h. It was concentrated in vacuo, treated with hexane and dried to yield 2.46 g (100%) of the title compound.
LRMS: m/z 160 (M+1)⁺
Retention time: 2.27 min (method5)
¹H NMR (200 MHz, DMSO-d₆) δ ppm 1.30 - 2.00 (m, 8 H) 4.50 (m, 3 H) 7.60 (d, 1H) 8.60 (s, 1 H)

### PREPARATION 22

### N-Cyclopentyl-5-(4-methoxy-3,5-dimethylphenyl)-1,3,4-thiadiazol-2-amine

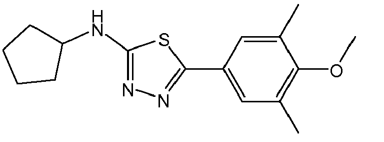

To a stirred solution of 4-methoxy-3,5-dimethylbenzoic acid (2.35 g, 13.04 mmol) in dioxane (20 ml) POCl₃ (1.4 ml, 15.29 mmol) was added. It was stirred at 90°C for 3h. It was allowed to cool down to room temperature and the title compound of Preparation 21 (2.46 g, 15.45 mmol) was added. It was stirred at 100°C for 2h. It was allowed to cool down to room temperature. It was poured onto water/ice and an aqueous 2M solution of NaOH was added to reach pH7. The solid obtained was filtered, washed and dried to yield 4.67 g (100%) of the title compound.
LRMS: m/z 304 (M+1)⁺
Retention time: 6.70 min (method B)
¹H NMR (200 MHz, DMSO-d₆) δ ppm 1.40 - 2.00 (m, 8 H) 2.30 (s, 6 H) 3.70 (s, 3H) 4.00 (m, 1 H) 7.40 (s, 2H) 8.20 (s, 1 H)

### PREPARATION 23

### N-Isopropylhydrazinecarbothioamide

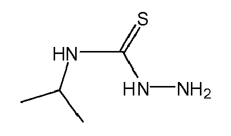

Obtained (99% yield) from 2-isothiocyanatopropane and hydrazine hydrate following the procedure described in Preparation 21.
LRMS: m/z 134 (M+1)⁺
Retention time: 3.18 min (method B)
¹H NMR (200 MHz, DMSO-d₆) δ ppm 1.10 (d, 6 H) 2.50 (s, 2 H) 4.40 (m, 1 H) 7.40 (m, 1 H) 9.20 (s, 1 H)

### PREPARATION 24

### N-Isopropyl-5-(4-methoxy-3,5-dimethylphenyl)-1,3,4-thiadiazol-2-amine

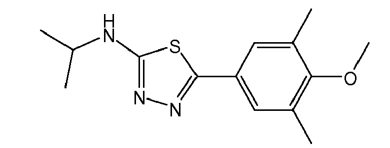

Obtained (56% yield) from 4-methoxy-3,5-dimethylbenzoic acid and the title compound of Preparation 23 following the procedure described in Preparation 22.
LRMS: m/z 278 (M+1)⁺
Retention time: 6.23 min (method B)
¹H NMR (200 MHz, DMSO-d₆) δ ppm 1.10 - 1.33 (m, 6 H) 2.13 - 2.36 (s, 6 H) 3.57 (s, 3 H) 3.77 - 3.97 (m, 1 H) 7.42 (s, 2 H) 7.79 (d, J=7.42 Hz, 1 H)

### PREPARATION 25

### 5-(4-Methoxy-3,5-dimethylphenyl)-N-(pyridin-4-ylmethyl)-1,3,4-thiadiazol-2-amine

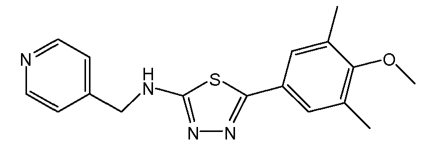

Obtained (15% yield) from isonicotinaldehyde and the title compound of Preparation 3 following the procedure described in Preparation 4 (using dichloromethane as extracting solvent and normal phase chromatography for the purification).
LRMS: m/z 327 (M+1)⁺
Retention time: 2.35 min (method A)
¹H NMR (200 MHz, CHLOROFORM-d) δ ppm 2.13 - 2.43 (m, 6 H) 3.74 (s, 3 H) 4.65 (s, 2 H) 7.15 - 7.40 (m, 4 H) 8.40 - 8.73 (m, 2 H)

### PREPARATION 26

### 4-[5-(Butylamino)-1,3,4-thiadiazol-2-yl]benzaldehyde

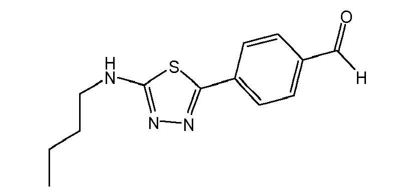

To a stirred solution of N-butyl-5-(4-vinylphenyl)-1,3,4-thiadiazol-2-amine (Preparation 7) (2.28 g, 8.79 mmol) in acetone/water (50 ml/10 ml) sodium periodate (5.65 g, 26.4 mmol) and osmium tetroxide (1.29 ml, 5.07 mmol) were added. It was stirred at room temperature for 3h. The mixture was filtered through celite and it was concentrated in vacuo. The residue was dissolved in ethyl acetate and washed with water. The organic phase was dried, filtered and concentrated in vacuo to give 1.76 g (83% purity) (64%) of the title compound.
LRMS: m/z 262 (M+1)⁺
Retention time: 5.87min (method B)
¹H NMR (200 MHz, CDCl₃) δ ppm 0.98 (t, *J*=7.22 Hz, 3 H) 1.46 (dd, *J*=15.03, 7.22 Hz, 2 H) 1.72 (t, *J*=3.51 Hz, 2 H) 3.41 (t, *J*=7.03 Hz, 2 H) 7.85 - 8.05 (m, 4 H) 10.04 (s, 1 H)

### PREPARATION 27

### 4-{5-[Butyl(2-chlorobenzyl)amino]-1,3,4-thiadiazol-2-yl}benzaldehyde

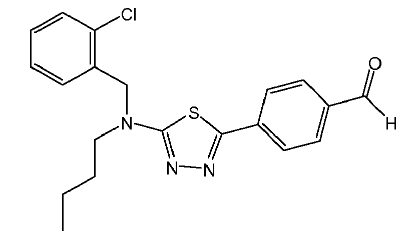

To a stirred solution of 4-(5-(butylamino)-1,3,4-thiadiazol-2-yl)benzaldehyde (Preparation 26) (0.3 g, 1.15 mmol) in DMF (4 ml) NaH (60%) (0.05 g, 2.29 mmol) was added. It was stirred at room temperature for 15 min. A solution of 1-(bromomethyl)-2-chlorobenzene (0.24 g, 1.14 mmol) in DMF (1 ml) was added dropwise. It was stirred at room temperature overnight. It was poured onto water/ice and extracted with ethyl acetate. The organic phase was dried, filtered and concentrated in vacuo. It was purified by reverse phase chromatography to give 0.15 g (34%) of the title compound.
LRMS: m/z 386 (M+1)⁺
Retention time: 3.83 min (method A)

### PREPARATION 28

### 4-{5-[Butyl(2,6-dichlorobenzyl)amino]-1,3,4-thiadiazol-2-yl}benzaldehyde

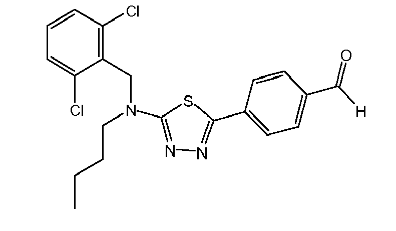

Obtained (72% yield) from 2-(bromomethyl)-1,3-dichlorobenzene and the title compound of Preparation 26 following the procedure described in Preparation 27 (treatment with hexane of the solid obtained from the concentration of the organic phase was done instead of a reverse phase purification).
LRMS: m/z 420 (M+1)⁺
Retention time: 7.55 min (method B)
¹H NMR (200 MHz, CHLOROFORM-d) δ ppm 0.66 - 1.02 (m, 3 H) 1.11 - 1.33 (m, 2 H) 1.44 - 1.77 (m, 2 H) 3.08 - 3.32 (m, 2 H) 5.05 (s, 2 H) 6.93 - 7.48 (m, 3 H) 7.73 - 8.14 (m, 4 H) 9.79 - 10.09 (m, 1 H)

### PREPARATION 29

### N-Butyl-N-(2-chlorobenzyl)-5-(4-{[(4S)-2,2-dimethyl-1,3-dioxolan-4-yl]methoxy}-3,5-dimethylphenyl)-1,3,4-thiadiazol-2-amine

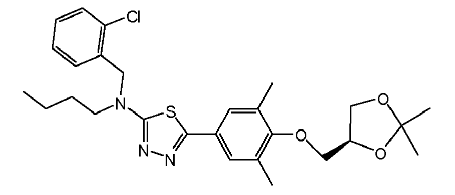

To a stirred solution of 4-(5-(butyl(2-chlorobenzyl)amino)-1,3,4-thiadiazol-2-yl)-2,6-dimethylphenol (title compound of example 27) (0.22 g, 0.55 mmol) in DMF (15 ml) NaH (60%) (0.06 g, 1.50 mmol) was added. It was stirred at 120°C for 30 min. A solution of (R)-4-(chloromethyl)-2,2-dimethyl-1,3-dioxolane (0.17 g, 1.10 mmol) and sodium iodine (0.17 g, 1.13 mmol) in DMF (5 ml) was added and it was stirred at 120°C for 48h. It was poured onto water/ice and extracted with ethylacetate. The organic phase was washed with water and brine, dried and concentrated in vacuo. It was purified by chromatography (SiO2, Hexane/ethyl acetate) to give 0.28 g (45%) of the title compound.
LRMS: m/z 516 (M+1)⁺
Retention time: 7.86 min (method B)

### PREPARATION 30

*N*-Butyl-N-(2-chlorobenzyl)-5-(4-{[(4R)-2,2-dimethyl-1,3-dioxolan-4-yl]methoxy}-3,5-dimethylphenyl)-1,3,4-thiadiazol-2-amine

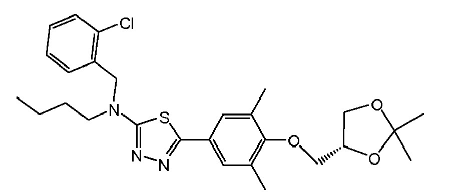

Obtained (21% yield) from (S)-4-(chloromethyl)-2,2-dimethyl-1,3-dioxolane and 4-(5-(butyl(2-chlorobenzyl)amino)-1,3,4-thiadiazol-2-yl)-2,6-dimethylphenol (the title compound of example 27) following the procedure described in Preparation 29.
LRMS: m/z 516 (M+1)⁺
Retention time: 7.96 min (method B)

### PREPARATION 31

### N-Butyl-N-(2-chlorobenzyl)-5-[3,5-dimethyl-4-(oxiran-2-ylmethoxy)phenyl]-1,3,4-thiadiazol-2-amine

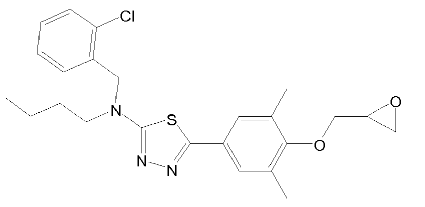

To a solution of 4-(5-(butyl(2-chlorobenzyl)amino)-1 ,3,4-thiadiazol-2-yl)-2,6-dimethylphenol (title compound of example 27) (0.11 g, 0.28 mmol) and oxiran-2-ylmethanol (0.025 ml, 0.38 mmol) in THF (0.34 ml) triphenylphosphine (0.097 g, 0.37 mmol) was added. Inert atmosphere was established and diisopropyl azodicarboxylate (0.073 ml, 0.37 mmol) was slowly added. It was stirred at 80°C in the microwave for 3h. It was concentrated in vacuo and the residue was disolved in dichloromethane. It was washed with water, dried and concentrated in vacuo. It was purified by chromatography (SiO2, Hexane/ethyl acetate) to give 0.064 g (50%) of the title compound.
LRMS: m/z 458 (M+1)⁺
Retention time: 7.91 min (method B)

### PREPARATION 32

### N-Ethyl-5-(4-vinylphenyl)-1,3,4-thiadiazol-2-amine

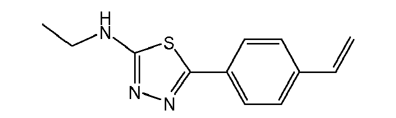

To a solution of 4-vinylbenzoic acid (18.62 g, 125.64 mmols) in dioxane ((100 ml) POCl₃ was added and it was heated at 90°C for 1 h. It was allowed to cool down to room temperature and N-ethylhydrazinecarbothioamide (13.68 g, 114.75 mmols) and it was stirred at reflux overnight. It was allowed to cool down to room temperature, water was added and it was concentrated in vacuo. To the solid obtained water and sodium hydroxide were added to reach pH 7-8. To the suspension obtained isopropanol was added. To the solution obtained diethyl ether was added. The solid obtained was filtered and dried to give 5.30 g (20%) of the title compound.
LRMS: m/z 232 (M+1)⁺
Retention time: 5.97 min (method B)

### PREPARATION 33

### N-Ethyl-N-[5-(4-vinylphenyl)-1,3,4-thiadiazol-2-yl]pyridin-2-amine

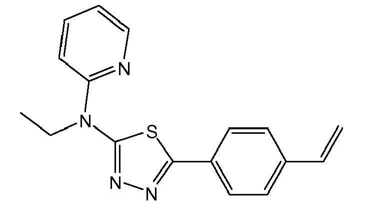

To a solution of N-ethyl-5-(4-vinylphenyl)-1,3,4-thiadiazol-2-amine (Preparation 32) (1.00 g, 4.32 mmols) in DMF (10 ml) under inert atmosphere 2-chloropyridine (0.37 ml, 3.91 mmols), BINAP (0.74 g, 1.18 mmols), Pd (AcO)₂ (0.19 g, 0.86 mmols) and Cs₂CO₃ (1.92 g, 5.90 mmols) were added. It was stirred in the microwave at 120°C for 3h. It was allowed to cool down to room temperature. The suspension obtained was filtered through celite and washed with ethyl acetate. It was purified by reverse phase chromatography to give 0.54 g of the title compound (40%).
LRMS: m/z 309 (M+1)⁺
Retention time: 7.45 min (method B)

### PREPARATION 34

### 4-{5-[Ethyl(pyridin-2-yl)amino]-1,3,4-thiadiazol-2-yl}benzaldehyde

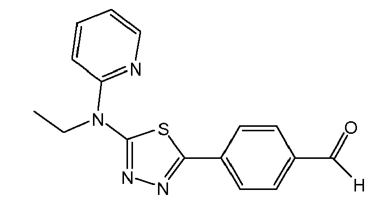

To a solution of N-ethyl-N-[5-(4-vinylphenyl)-1,3,4-thiadiazol-2-yl]pyridin-2-amine (Preparation 33) (0.54 g, 1.74 mmols) in acetone/water (10 ml/1 ml) osmium tetroxide (0.05 ml, 0.75 mmols) and sodium periodate (1.19 g, 5.23 mmols) were added. It was stirred at room temperature overnight. The suspension obtained was filtered through celite and it was concentrated in vacuo. Ethyl acetate was added and it was washed with water. The organic phase was dried, filtered and concentrated in vacuo without further purification to give 0.37 (76% purity) g of the title compund (52%).
LRMS: m/z 311 (M+1)⁺
Retention time: 6.50 min (method B)

### PREPARATION 35

### N-Butyl-N-[5-(4-vinylphenyl)-1,3,4-thiadiazol-2-yl]pyridin-2-amine

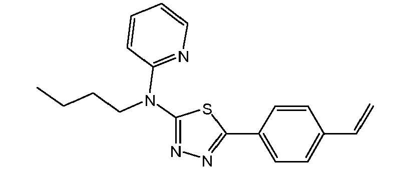

Obtained (22%) from N-butyl-5-(4-vinylphenyl)-1,3,4-thiadiazol-2-amine (Preparation 7) and 2-chloropyridine following the procedure described in Preparation 33.
LRMS: m/z 337 (M+1)⁺
Retention time: 7.70 min (method B)

### PREPARATION 36

### 4-{5-[butyl(pyridin-2-yl)amino]-1,3,4-thiadiazol-2-yl}benzaldehyde

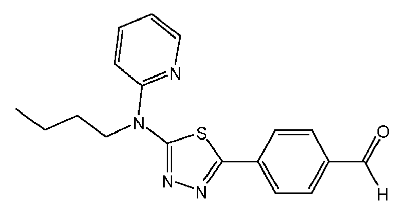

Obtained (82%) from N-butyl-N-[5-(4-vinylphenyl)-1,3,4-thiadiazol-2-yl]pyridin-2-amine (Preparation 35) following the procedure described in Preparation 34.
LRMS: m/z 339 (M+1)⁺
Retention time: 7.15 min (method B)

### PREPARATION 37

### N-Butyl-N-[5-(4-formylphenyl)-1,3,4-thiadiazol-2-yl]-1-phenylmethanesulfonamide

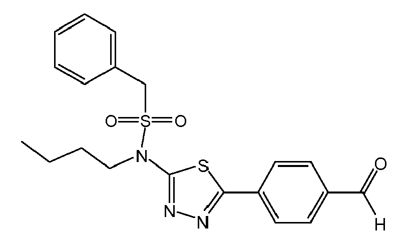

To a solution of 4-[5-(butylamino)-1,3,4-thiadiazol-2-yl]benzaldehyde (Preparation 26) ( 0.30 g, 1.15 mmols) in DMF (10 ml) sodium hydride was added. It was stirred at room temperature for 30 minutes. Under inert atmosphere benzylsulfonyl chloride (0.262 g, 1.38 mmols) was added and it was stirred at room temperature for 4h. More benzylsulfonylchloride (0.200 g) was added and it was stirred under the previous conditions overnight. A 2N aqueous solution of HCl and water were added and it was extracted with ethylacetate. The organic phase was dried, filtered and concentrated in vacuo. It was purified by reverse phase chromatography to give 0.28 g (59 % yield) of the title compound.
LRMS: m/z 414 (M-1)⁺
Retention time: 6.95 min (method B)

### PREPARATION 38

### N-Butyl-N-[5-(4-formylphenyl)-1,3,4-thiadiazol-2-yl]benzenesulfonamide

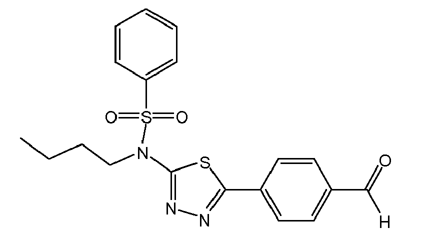

Obtained (41 %) from 4-[5-(butylamino)-1,3,4-thiadiazol-2-yl]benzaldehyde (Preparation 26) and phenylsulfonylchloride following the procedure described in Preparation 37.
LRMS: m/z 402 (M+1)⁺
Retention time: 7.10 min (method B)

### PREPARATION 39

### 4-[5-(Ethylamino)-1,3,4-thiadiazol-2-yl]benzaldehyde

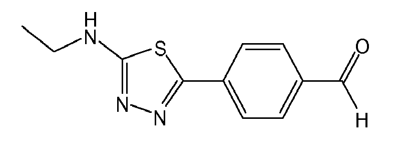

Obtained (22%) from N-ethyl-5-(4-vinylphenyl)-1,3,4-thiadiazol-2-amine (Preparation 32) following the procedure described in Preparation 34.
LRMS: m/z 234 (M+1)⁺
Retention time: 5.00 min (method B)

### PREPARATION 40

### N-Ethyl-N-[5-(4-formylphenyl)-1,3,4-thiadiazol-2-yl]-1-phenylmethanesulfonamide

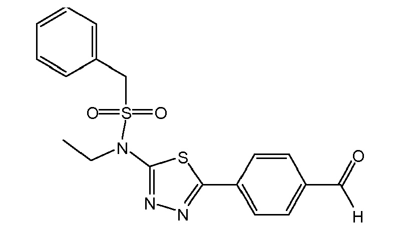

Obtained (27%) from 4-[5-(ethylamino)-1,3,4-thiadiazol-2-yl]benzaldehyde (Preparation 39) following the procedure described in Preparation 37 but using THF instead of DMF as solvent.
LRMS: m/z 388 (M+1)⁺
Retention time: 6.40 min (method B)

### EXAMPLES

### EXAMPLE 1

### N-Butyl-N-(2-chlorobenzyl)-5-(4-methoxy-3,5-dimethylphenyl)-1,3,4-thiadiazol-2-amine

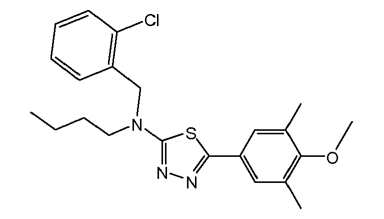

To a stirred solution of the title compound of Preparation 2 (0.10 g, 0.34 mmol) in DMF (1 ml) sodium hydride (60%) (0.016 g, 0.67 mmol) was added. It was stirred at room temperature for 30 min. A solution of 1-(bromomethyl)-2-chlorobenzene (0.078 g, 0.38 mmol) in DMF (1 ml) was added dropwise. It was stirred at room temperature for 5 days. A few drops of water were added to the mixture and it was directly purified by reverse phase chromatography. 0.016 g (11% yield) of the title compound were obtained.
LRMS: m/z 416 (M+1)⁺
Retention time: 21.1 min (method C)
¹H NMR (200 MHz, DMSO-*d*₆) δ ppm 0.89 (t, *J*=7.22 Hz, 3 H) 1.14 - 1.42 (m, 2 H) 1.54-1.70 (m, 2H) 2.26 (s, 6 H) 3.42 - 3.60 (m, 2 H) 3.68 (s, 3 H) 4.81 (s, 2 H) 7.23 - 7.38 (m, 3 H) 7.44 (s, 2 H) 7.48 - 7.61 (m, 1 H)

### EXAMPLE 2

### N-Benzyl-N-butyl-5-(4-methoxy-3,5-dimethylphenyl)-1,3,4-thiadiazol-2-amine

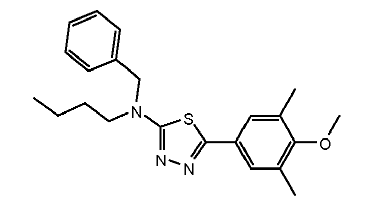

Obtained (51% yield) from (bromomethyl)benzene and the title compound of Preparation 2 following the procedure described in example 1. The reaction mixture obtained was poured onto water and extracted with dichloromethane. The organic phase was dried and concentrated in vacuo. The residue was purified by reverse phase chromatography.
LRMS: m/z 382 (M+1)⁺
Retention time: 20.4 min (method C)
¹H NMR (200 MHz, DMSO-*d*₆) δ ppm 0.87 (t, 3 H) 1.16 - 1.42 (m, 2 H) 1.50 - 1.74 (m, 2 H) 2.18 - 2.31 (s, 6 H) 3.40 - 3.57 (m, 2 H) 3.68 (s, 3 H) 4.74 (s, 2 H) 7.22 - 7.40 (m, 5 H) 7.41 - 7.47 (m, 2 H)

### EXAMPLE 3

### N-Butyl-N-(2-fluorobenzyl)-5-(4-methoxy-3,5-dimethylphenyl)-1,3,4-thiadiazol-2-amine

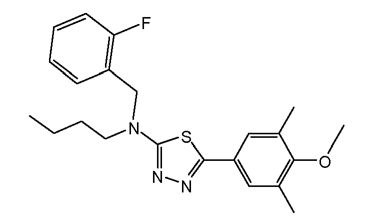

Obtained (58% yield) from 1-(bromomethyl)-2-fluorobenzene and the title compound of Preparation 2 following the procedure described in example 2 but using ethyl acetate as extracting solvent.
LRMS: m/z 400 (M+1)⁺
Retention time: 20.5 min (method C)
¹H NMR (200 MHz, DMSO-*d*₆) δ ppm 0.89 (t, *J*=7.22 Hz, 3 H) 1.14 - 1.42 (m, 2H) 1.49 - 1.77 (m, 2 H) 2.26 (s, 6 H) 3.42 - 3.56 (m, 2 H) 3.68 (s, 3 H) 4.79 (s, 2 H) 7.08 - 7.27 (m, 2 H) 7.27 - 7.49 (m, 4 H)

### EXAMPLE 4

### N-Butyl-N-(2,6-difluorobenzyl)-5-(4-methoxy-3,5-dimethylphenyl)-1,3,4-thiadiazol-2-amine

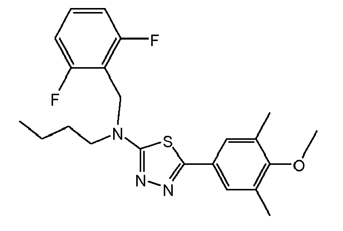

Obtained (47% yield) from 2-(bromomethyl)-1,3-difluorobenzene and the title compound of Preparation 2 following the procedure described in example 3.
LRMS: m/z 418 (M+1)⁺
Retention time: 20.4 min (method C)
¹H NMR (200 MHz, DMSO-*d*₆) δ ppm 0.86 (t, *J*=7.22 Hz, 3 H) 1.11 - 1.39 (m, 2 H) 1.44 - 1.69 (m, 2 H) 2.27 (s, 6 H) 3.40 (t, *J*=7.03 Hz, 2 H) 3.69 (s, 3 H) 4.80 (s, 2 H) 7.03 - 7.24 (m, 2 H) 7.31 - 7.58 (m, 3 H)

### EXAMPLE 5

### N-Butyl-N-(2,4-difluorobenzyl)-5-(4-methoxy-3,5-dimethylphenyl)-1,3,4-thiadiazol-2-amine

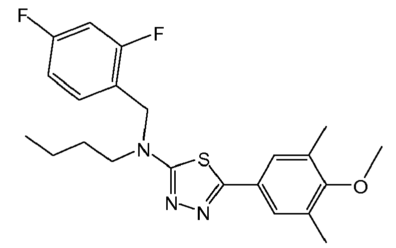

Obtained (34% yield) from 1-(bromomethyl)-2,4-difluorobenzene and the title compound of Preparation 2 following the procedure described in example 3.
LRMS: m/z 418 (M+1)⁺
Retention time: 20.5 min (method C) ¹H NMR (200 MHz, DMSO-*d*₆) δ ppm 0.89 (t, *J*=7.22 Hz, 3 H) 1.12 - 1.41 (m, 2H) 1.46 - 1.80 (m, 2 H) 2.26 (s, 6 H) 3.37 - 3.52 (m, 2 H) 3.69 (s, 3 H) 4.75 (s, 2 H) 6.95 - 7.23 (m, *J*=1.56 Hz, 1 H) 7.20 - 7.62 (m, 4 H)

### EXAMPLE 6

### N-Butyl-N-(2,6-dichlorobenzyl)-5-(4-methoxy-3,5-dimethylphenyl)-1,3,4-thiadiazol-2-amine

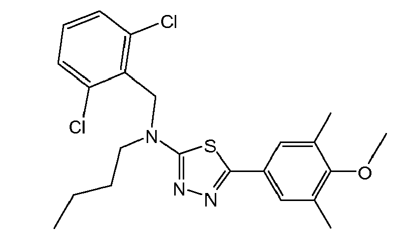

Obtained (44% yield) from 2-(bromomethyl)-1,3-dichlorobenzene and the title compound of Preparation 2 following the procedure described in example 3.
LRMS: m/z 450 (M+1)⁺
Retention time: 21.4 min (method C)
¹H NMR (200 MHz, DMSO-*d*₆) δ ppm 0.79 (t, *J*=7.22 Hz, 3 H) 1.01 - 1.29 (m, 2 H) 1.34 - 1.59 (m, *J*=7.03 Hz, 2 H) 2.27 (s, 6 H) 3.21 (t, *J*=14.45 Hz, 2 H) 3.69 (s, 3 H) 4.97 (s, 2 H) 7.38 - 7.52 (m, 3 H) 7.47 - 7.64 (m, 2 H)

### EXAMPLE 7

### 2-({Butyl[5-(4-methoxy-3,5-dimethylphenyl)-1,3,4-thiadiazol-2-yl]amino}methyl)-benzonitrile

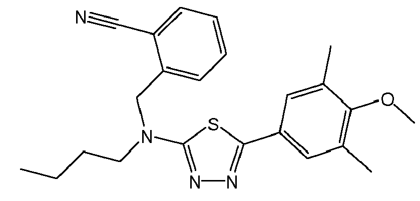

Obtained (49% yield) from 2-(bromomethyl)benzonitrile and the title compound of Preparation 2 following the procedure described in example 3 but using THF as reaction solvent. Normal phase chromatography was used to purify the final product.
LRMS: m/z 407 (M+1)⁺
Retention time: 19.4 min (method C)
¹H NMR (200 MHz, CHLOROFORM-*d*) δ ppm 0.95 (t, *J*=7.22 Hz, 3 H) 1.20 - 1.52 (m, *J*=14.84, 7.42 Hz, 2 H) 1.55 - 1.89 (m, 2 H) 2.31 (s, 6 H) 3.51 (t, 2 H) 3.74 (s, 3 H) 5.00 (s, 2 H) 7.34 - 7.53 (m, 3 H) 7.53 - 7.79 (m, 3 H)

### EXAMPLE 8

### N-Butyl-N-(3-methoxybenzyl)-5-(4-methoxy-3,5-dimethylphenyl)-1,3,4-thiadiazol-2-amine

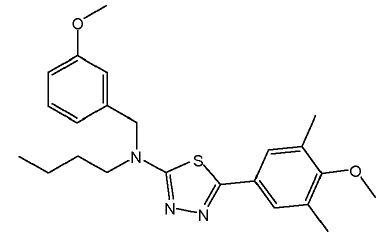

Obtained (57% yield) from 1-(chloromethyl)-3-methoxybenzene and the title compound of Preparation 2 following the procedure described in example 7 (reaction temperature: 60°C).
LRMS: m/z 412 (M+1)⁺
Retention time: 20.3 min (method C)
¹H NMR (200 MHz, CHLOROFORM-*d*) δ ppm 0.94 (t, *J*=7.22 Hz, 3 H) 1.14 - 1.53 (m, 2 H) 1.52 - 1.84 (m, 2 H) 2.31 (s, 6 H) 3.48 (t, 2 H) 3.74 (s, 3 H) 3.79 (s, 3 H) 4.72 (s, 2 H) 6.67 - 7.02 (m, 4 H) 7.45 (s, 2 H)

### EXAMPLE 9

### N-(2-Chlorobenzyl)-N-(cyclopropylmethyl)-5-(4-methoxy-3,5-dimethylphenyl)-1,3,4-thiadiazol-2-amine

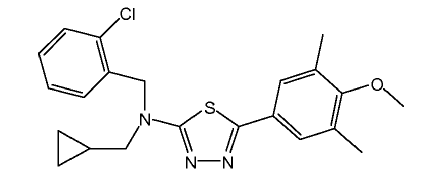

To a stirred solution of the title compound of Preparation 4 (0.20 g, 0.56 mmol) in DMF (4 ml) sodium hydride (60%) (0.045 g, 1.13 mmol) was added. It was stirred at room temperature for 30 min. (Bromomethyl)cyclopropane (0.15 g, 1.11 mmol) was added dropwise and it was stirred at room temperature overnight. The mixture was poured onto water and extracted with ethyl acetate. The organic phase was washed and dried. It was purified chromatography (SiO₂, Hexane/ethyl acetate 8:2) to yield 0.11 g (46%) of the title compound.
LRMS: m/z 414 (M+1)⁺
Retention time: 20.6 min (method C)
1 H NMR (200 MHz, CHLOROFORM-*d*) δ ppm 0.09 - 0.38 (m, 2 H) 0.40 - 0.71 (m, 2 H) 0.97 - 1.43 (m, 1 H) 2.30 (s, 6 H) 3.50 (d, 2H) 3.73 (s, 3 H) 4.93 (s, 2 H) 7.29 - 7.55 (m, 6 H)

### EXAMPLE 10

### N-(2-Chlorobenzyl)-5-(4-methoxy-3,5-dimethylphenyl)-N-(2-methoxyethyl)-1,3,4-thiadiazol-2-amine

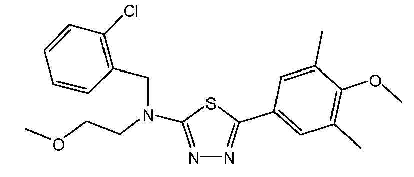

Obtained (17% yield) from 1-bromo-2-methoxyethane and the title compound of Preparation 4 following the procedure described in example 9.
LRMS: m/z 418 (M+1)⁺
Retention time: 19.4 min (method C)
¹H NMR (200 MHz, CHLOROFORM-*d*) δ ppm 2.30 (s, 6 H) 3.34 (s, 3 H) 3.56 - 3.75 (m, 5 H) 3.70 - 3.89 (m, 2 H) 4.88 (s, 2 H) 7.00 - 7.37 (m, 4 H) 7.32 - 7.53 (m, 2 H)

### EXAMPLE 11

### N-(2-Chlorobenzyl)-5-(4-methoxy-3,5-dimethylphenyl)-N-propyl-1,3,4-thiadiazol-2-amine

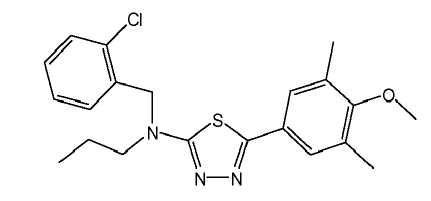

Obtained (70% yield) from 1-bromopropane and the title compound of Preparation 4 following the procedure described in example 10 (2 eq of NaH (60%) were used and the reaction temperature was 60°C in this case).
LRMS: m/z 402 (M+1)⁺
Retention time: 20.5 min (method C)
¹H NMR (200 MHz, CHLOROFORM-*d*) δ ppm 0.96 (t, *J*=7.42 Hz, 3 H) 1.65 - 1.90 (m, *J*=7.81 Hz, 2 H) 2.30 (s, 6 H) 3.49 (t, 2 H) 3.73 (s, 3 H) 4.85 (s, 2 H) 6.96 - 7.36 (m, 3 H) 7.32 - 7.55 (m, 3 H)

### EXAMPLE 12

### N-(2-Chlorobenzyl)-5-(4-methoxy-3,5-dimethylphenyl)-N-(4,4,4-trifluorobutyl)-1,3,4-thiadiazol-2-amine

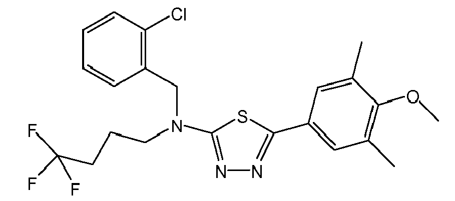

Obtained (28% yield) from 4-bromo-1,1,1-trifluorobutane and the title compound of Preparation 4 following the procedure described in example 11.
LRMS: m/z 470 (M+1)⁺
Retention time: 20.45 min (method C)
1 H NMR (200 MHz, CHLOROFORM-*d*) δ ppm 1.83 - 2.10 (m, 2 H) 2.09 - 2.23 (m, 2 H) 2.18 - 2.40 (s, 6 H) 3.46 - 3.71 (m, 2 H) 3.68 - 3.79 (s, 3 H) 4.79 (s, 2H) 7.11 - 7.39 (m, 3 H) 7.39 - 7.58 (m, 3 H)

### EXAMPLE 13

### N-(2-Chlorobenzyl)-N-cyclopentyl-5-(4-methoxy-3,5-dimethylphenyl)-1,3,4-thiadiazol-2-amine

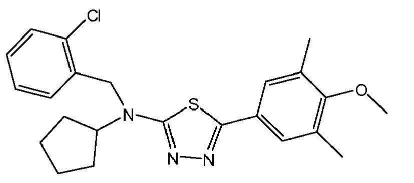

Obtained (25% yield) from 1-(bromomethyl)-2-chlorobenzene and the title compound of Preparation 22 following the procedure described in example 11.
LRMS: m/z 428 (M+1)⁺
Retention time: 22.0 min (method C)
¹H NMR (200 MHz, CHLOROFORM-*d*) δ ppm 0.62 - 0.98 (m, 2 H) 1.14 - 1.36 (m, 2 H) 1.58 (s, 4 H) 2.17 - 2.42 (s, 6 H) 3.30 (m, 1 H) 3.73 (s, 3 H) 4.66 (d, *J*=23.43 Hz, 2 H) 6.99 - 7.30 (m, 3 H) 7.34 - 7.59 (m, 3 H)

### EXAMPLE 14

### N-(2-Chlorobenzyl)-N-isopropyl-5-(4-methoxy-3,5-dimethylphenyl)-1,3,4-thiadiazol-2-amine

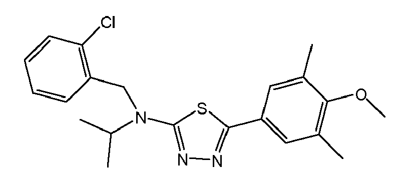

Obtained (18% yield) from 1-(bromomethyl)-2-chlorobenzene and the title compound of Preparation 24 following the procedure described in example 11.
LRMS: m/z 402 (M+1)⁺
Retention time: 21.1 min (method C)

### EXAMPLE 15

### N-Butyl-N-(2-fluorobenzyl)-5-(2-methylpyridin-4-yl)-1,3,4-thiadiazol-2-amine

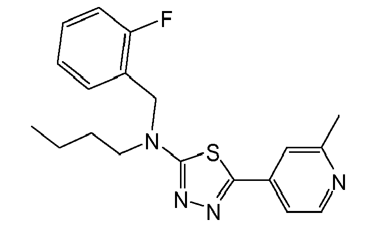

Obtained (43% yield) from 1-(bromomethyl)-2-fluorobenzene and the title compound of Preparation 5 following the procedure described in example 3 (1 equivalent of NaH (60%) was used in this case).
LRMS: m/z 357 (M+1)⁺
Retention time: 15.9 min (method C)
¹H NMR (200 MHz, CHLOROFORM-*d*) δ ppm 0.95 (t, *J*=7.22 Hz, 3 H) 1.11 - 1.50 (m, 2 H) 1.48 - 1.83 (m, 2 H) 2.60 (s, 3 H) 3.16 - 3.60 (m, 2 H) 4.84 (s, 2 H) 6.85 - 7.19 (m, 3 H) 7.33 - 7.62 (m, 3 H) 8.53 (d, *J*=5.47 Hz, 1 H)

### EXAMPLE 16

### N-Butyl-N-(2,6-dichlorobenzyl)-5-(2-methylpyridin-4-yl)-1,3,4-thiadiazol-2-amine

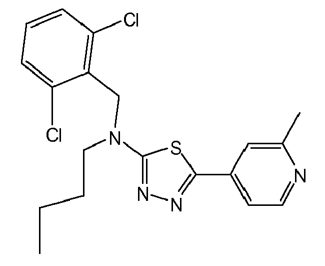

Obtained (45% yield) from 2-(bromomethyl)-1,3-dichlorobenzene and the title compound of Preparation 5 following the procedure described in example 15.
LRMS: m/z 407 (M+1)⁺
Retention time: 18.2 min (method C)
¹H NMR (200 MHz, CHLOROFORM-*d*) δ ppm 0.86 (t, *J*=7.22 Hz, 3 H) 1.07 - 1.42 (m, 2 H) 1.39 - 1.75 (m, 2 H) 2.62 (s, 3 H) 3.00-3.49 (m, 2 H) 5.10 (s, 2 H) 7.11 - 7.40 (m, 3 H) 7.44 - 7.68 (m, 2 H) 8.55 (d, *J*=5.08 Hz, 1 H)

### EXAMPLE 17

### N-Butyl-N-(cyclobutylmethyl)-5-(2-methylpyridin-4-yl)-1,3,4-thiadiazol-2-amine

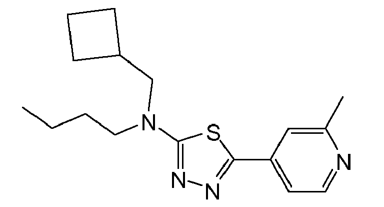

Obtained (40% yield) from (bromomethyl)cyclobutane and the title compound of Preparation 5 following the procedure described in example 15.
LRMS: m/z 317 (M+1)⁺
Retention time: 15.5 min (method C)
¹H NMR (200 MHz, CHLOROFORM-*d*) δ ppm 0.97 (t, *J*=7.22 Hz, 3 H) 1.15 - 1.50(m,2H)1.52-1.75(m,2H)1.72-1.91 (m, 4 H) 1.74 -1.90 (m, 2 H) 2.46 - 2.67 (s, 3 H) 2.71 - 2.94 (m, 1 H) 3.33 - 3.81 (m, 4 H) 7.36 - 7.64 (m, 2 H) 8.53 (d, *J*=5.08 Hz, 1 H)

### EXAMPLE 18

### N-Butyl-5-(2-methylpyridin-4-yl)-N-(2-phenylethyl)-1,3,4-thiadiazol-2-amine

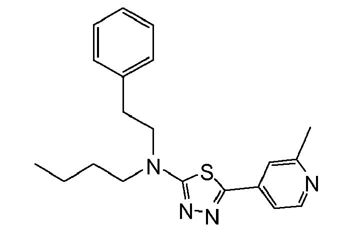

Obtained (10% yield) from (2-bromoethyl)benzene and the title compound of Preparation 5 following the procedure described in example 15.
LRMS: m/z 353 (M+1)⁺
Retention time: 16.0 min (method C)
¹H NMR (200 MHz, CHLOROFORM-d) δ ppm 0.83 (t, 3 H) 1.07 - 1.43 (m, 2 H) 1.45 - 1.76 (m, 2 H) 2.55 (s, 3 H) 2.78 - 3.06 (m, 2 H) 3.19 - 3.38 (m, 2 H) 3.49 - 3.76 (m, 2 H) 6.96 - 7.35 (m, 5 H) 7.33 - 7.61 (m, 2 H) 8.47 (d, J=5.08 Hz, 1 H)

### EXAMPLE 19

### 5-(4-Methoxy-3,5-dimethylphenyl)-N-propyl-N-(pyridin-4-ylmethyl)-1,3,4-thiadiazol-2-amine

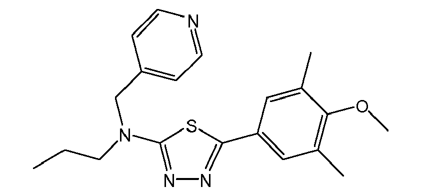

Obtained (29% yield) from 1-bromopropane and the title compound of Preparation 25 following the procedure described in example 1 (reaction temperature was 50°C and normal phase chromatography was used in this case).
LRMS: m/z 369 (M+1)⁺
Retention time: 14.2 min (method C)
¹H NMR (200 MHz, CHLOROFORM-*d*) δ ppm 0.96 (t, J=7.42 Hz, 3 H) 1.05 - 1.35 (m, 2 H) 2.31 (s, 6 H) 3.26 - 3.56 (m, 2 H) 3.74 (s, 3 H) 4.79 (s, 2 H) 6.92 - 7.25 (m, 3 H) 7.39 - 7.55 (m, 1 H) 8.57 (d, J=6.25 Hz, 2 H)

### EXAMPLE 20

### N-Butyl-N-(2,6-dichlorobenzyl)-5-imidazo[1,2-a]pyridin-6-yl-1,3,4-thiadiazol-2-amine

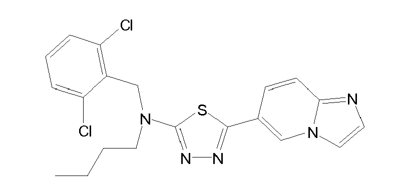

Obtained (31% yield) from 2-(bromomethyl)-1,3-dichlorobenzene and the title compound of Preparation 6 following the procedure described in example 15.
LRMS: m/z 432 (M+1)⁺
Retention time: 13.7 min (method C)
¹H NMR (200 MHz, DMSO-d₆) δ ppm 0.80 (t, J=7.22 Hz, 3 H) 0.97 - 1.31 (m, 2H) 1.35-1.67 (m, 2 H) 3.01 - 3.29 (m, 2 H) 5.00 (s, 2 H) 7.17-7.78 (m, 6 H) 8.02 (s, 1H) 9.12 (s, 1 H)

### EXAMPLE 21

### N-Butyl-N-(2-chlorobenzyl)-5-imidazo[1,2-a]pyridin-6-yl-1,3,4-thiadiazol-2-amine

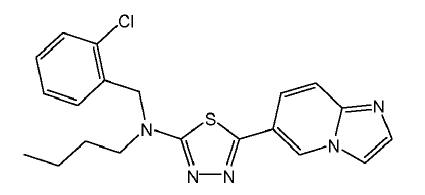

Obtained (31% yield) from 1-(bromomethyl)-2-chlorobenzene and the title compound of Preparation 6 following the procedure described in example 15.
LRMS: 398 m/z (M+1)⁺
Retention time: 13.2 min (method C)
¹H NMR (200 MHz, CHLOROFORM-d) δ ppm 0.46 - 1.05 (m, 3 H) 1.16 - 1.51 (m, 2 H) 1.58 - 1.86 (m, J=7.03 Hz, 2 H) 3.38 - 3.92 (m, 2 H) 4.86 (s, 2 H) 7.11 - 7.50 (m, 4 H) 7.48 - 7.84 (m, 4 H) 8.61 (s, 1 H)

### EXAMPLE 22

### 1-(4-{5-[Butyl(2-chlorobenzyl)amino]-1,3,4-thiadiazol-2-yl}benzyl)azetidine-3-carboxylic acid

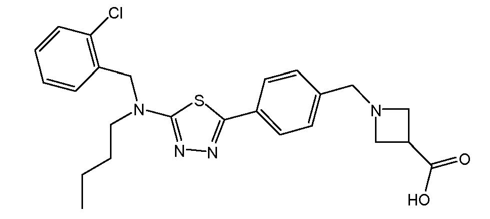

To a stirred suspension of 4-(5-(butyl(2-chlorobenzyl)amino)-1,3,4-thiadiazol-2-yl)benzaldehyde (Preparation 27) (0.15 g, 0.39 mmol) in methanol (2 ml) azetidine-3-carboxylic acid (0.043 g, 0.43 mmol) and acetic acid (0.2 ml, 3.50 mmol) were added. It was stirred at room temperature overnight. Sodium cyanoborohydride (0.015 g, 0.24 mmol) were added and it was stirred at room temperature for 1h. The mixture was concentrated in vacuo. Ethyl acetate was added. It was washed with water, dried, filtered and concentrated in vacuo. It was purified by reverse phase chromatography to yield 0.025 g (22%) of the title compound.
LRMS: m/z 471 (M+1)⁺
Retention time: 14.8 min (method C)
¹H NMR (200 MHz, DMSO-d₆) δ ppm 0.89 (t, J=7.22 Hz, 3 H) 1.16 - 1.45 (m, 2H) 1.43 - 1.80 (m, 2 H) 2.98 - 3.29 (m, 4 H) 3.00 - 3.29 (m, 2 H) 3.29 - 3.63 (m, 3 H) 4.82 (s, 2 H) 7.15 - 7.42 (m, 5 H) 7.44 - 7.59 (m, 1 H) 7.69 (d, J=8.20 Hz, 2H)

### EXAMPLE 23

### 1-(4-{5-[Butyl(2,6-dichlorobenzyl)amino]-1,3,4-thiadiazol-2-yl}benzyl)azetidine-3-carboxylic acid

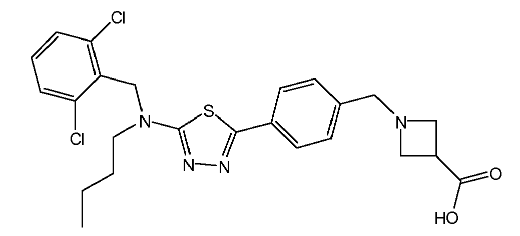

Obtained (9% yield) from azetidine-3-carboxylic acid and the title compound of Preparation 28 following the procedure described in example 22.
LRMS: m/z 505 (M+1)⁺ Retention time: 15.1 min (method C)
1 H NMR (200 MHz, DMSO-d₆) δ ppm 0.79 (t, J=7.22 Hz, 3 H) 1.04 - 1.31 (m, 2H) 1.32 - 1.65 (m, 2 H) 2.98 - 3.30 (m, 6 H) 3.31 - 3.50 (m, 3 H) 4.98 (s, 2 H) 7.26 - 7.49 (m, 3 H) 7.52 - 7.62 (m, 2 H) 7.72 (d, J=8.20 Hz, 2 H)

### EXAMPLE 24

### N-Butyl-5-(2-chloro-6-methoxypyridin-4-yl)-N-(2-fluorobenzyl)-1,3,4-thiadiazol-2-amine

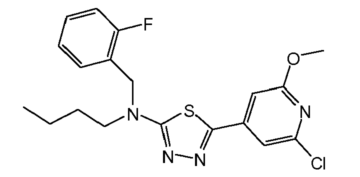

Obtained (64% yield) from 1-(bromomethyl)-2-fluorobenzene and the title compound of Preparation 8 following the procedure described in example 3 (2 equivalent of alquilation agent was used in this case).
LRMS: m/z 407 (M+1)⁺
Retention time: 20.8 min (method C)
¹H NMR (200 MHz, DMSO-d₆) δ ppm 0.89 (t, J=7.22 Hz, 3 H) 1.09 - 1.38 (m, 2H) 1.46 - 1.73 (m, 2 H) 3.40 - 3.65 (m, 2 H) 3.89 (s, 3 H) 4.83 (s, 2 H) 6.93 - 7.26 (m, 3 H) 7.24 - 7.57 (m, 3 H)

### EXAMPLE 25

### N-Butyl-N-(2-fluorobenzyl)-5-(2-methoxypyridin-4-yl)-1,3,4-thiadiazol-2-amine

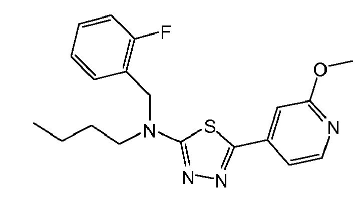

To a stirred solution of the title componud of example 24 (0.45 g, 1.11 mmol) in methanol (100 ml) Pd/C (10%) (0.12 g, 0.11 mmol) was added. It was stirred under hydrogen at 35 psi and room temperature overnight. 4M solution of HCl in dioxane (0.2 ml) was added and stirred the mixture was stirred under hydrogen at 40 psi and room temperature overnight. More 4M solution of HCl/dioxane (0.2 ml) was added and stirred at 40 psi of hydrogen at room temperature for three days. Triethylamine (0.5 ml) was added and it was maintained in the hydrogenator for 24h under the previous conditions. The catalyst was filtered and the filtrate was concentrated in vacuo. It was purified by chromatography (SiO₂, Hexane/ethylacetate) to give 0.41 g (23%) of the title compound.
LRMS: m/z 373 (M+1)⁺
Retention time: 19.3 min (method C)
¹H NMR (200 MHz, CHLOROFORM-d) δ ppm 0.95 (t, J=7.22 Hz, 3 H) 1.21 - 1.45 (m, 2 H) 1.60 - 1.86 (m, 2 H) 3.26 - 3.62 (m, 2 H) 3.97 (s, 3 H) 4.84 (s, 2 H) 6.73 - 7.21 (m, 3 H) 7.29 - 7.60 (m, 3 H) 8.19 (d, J=5.47 Hz, 1 H)

### EXAMPLE 26

### N-Butyl-N-(2,6-dichlorobenzyl)-5-(6-methoxypyridin-3-yl)-1,3,4-thiadiazol-2-amine

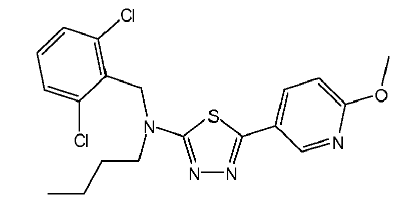

Obtained (37% yield) from 2-(bromomethyl)-1,3-dichlorobenzene and the title compound of Preparation 10 following the procedure described in example 15.
LRMS: m/z 423 (M+1)⁺
Retention time: 20.4 min (method C)
¹H NMR (200 MHz, CHLOROFORM-d) δ ppm 0.85 (t, J=7.22 Hz, 3 H) 1.14 - 1.42 (m, 2 H) 1.42 - 1.83 (m, 2 H) 2.98 - 3.34 (m, 2 H) 3.98 (s, 3 H) 4.76 - 5.23 (m, 2 H) 6.81 (d, J=8.59 Hz, 1 H) 7.08 - 7.51 (m, 3 H) 8.12 (dd, J=8.59, 2.34 Hz, 1 H) 8.51 (d, J=1.56 Hz, 1 H)

### EXAMPLE 27

### 4-{5-[Butyl(2-chlorobenzyl)amino]-1,3,4-thiadiazol-2-yl}-2,6-dimethylphenol

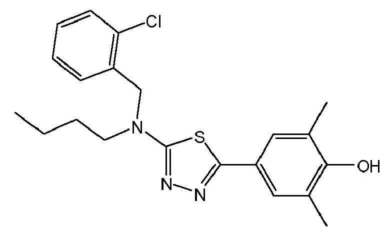

Obtained (79% yield) from the title compound of example 1 following the procedure described in Preparation 9.
LRMS: m/z 402 (M+1)⁺
Retention time: 19.8 min (method C)
¹H NMR (200 MHz, CHLOROFORM-d) δ ppm 0.94 (t, J=7.22 Hz, 3 H) 1.18 - 1.50 (m, J=15.03, 7.22 Hz, 2 H) 1.58 - 1.82 (m, 2 H) 2.27 (s, 6 H) 3.16 - 3.70 (m, 2 H) 4.73 - 4.93 (m, 2 H) 4.93 (s, 1 H) 7.13 - 7.31 (m, 3 H) 7.31 - 7.48 (m, 3H)

### EXAMPLE 28

### N-Butyl-N-(2,6-dichlorobenzyl)-5-imidazo[1,2-a]pyridin-7-yl-1,3,4-thiadiazol-2-amine

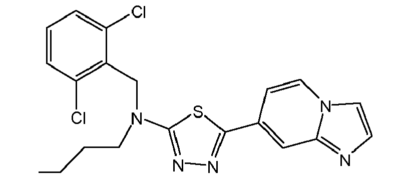

Obtained (63% yield) from 2-(bromomethyl)-1,3-dichlorobenzene and the title compound of Preparation 12 following the procedure described in example 15.
LRMS: m/z 432 (M+1)⁺
Retention time: 13.3 min (method C)
¹H NMR (200 MHz, DMSO-d₆) δ ppm 0.80 (t, J=7.22 Hz, 3 H) 1.05 - 1.35 (m, J=7.42 Hz, 2 H) 1.38 - 1.66 (m, 2 H) 3.06 - 3.41 (m, 2 H) 5.01 (s, 2 H) 7.32 - 7.74 (m, 5 H) 7.80 - 7.89 (m, 1 H) 8.04 (s, 1 H) 8.62 (d, J=7.42 Hz, 1 H)

### EXAMPLE 29

### (2R)-3-(4-{5-[Butyl(2-chlorobenzyl)amino]-1,3,4-thiadiazol-2-yl}-2,6-dimethyl-phenoxy)propane-1,2-diol

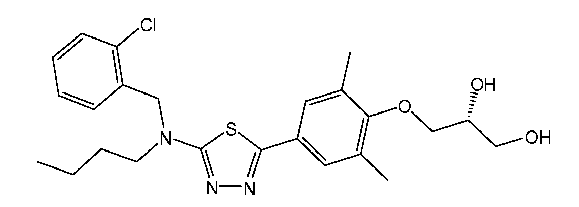

To a stirred solution of title compound of Preparation 29 (0.15 g, 0.29 mmol) in acetonitrile (5 ml) a 2N aqueous solution of HCl (1.3 ml) was added. It was stirred at room temperature for 2h. It was concentrated in vacuo and the residue was dissolved in water. A saturated aqueous solution of sodium hydrogen carbonate was added to reach pH 8-9. It was extracted with ethylacetate, washed with brine, dried and concentrated in vacuo to give 0.042 g (30%) of the title compound.
LRMS: m/z 476 (M+1)⁺
Retention time: 18.6 min (method C)
¹H NMR (200 MHz, DMSO-d₆) δ ppm 0.89 (t, 3 H) 1.12 - 1.44 (m, J=14.84, 7.03 Hz, 2 H) 1.48 - 1.63 (m, 2 H) 2.27 (s, 6H) 3.19 - 3.39 (m, 4 H) 3.41 - 3.54 (m, J=5.66, 5.66 Hz, 3 H) 3.61 - 3.74 (m, 1 H) 3.74 - 3.87 (m, 1 H) 4.81 (s, 2 H) 7.27
- 7.40 (m, 3 H) 7.43 (s, 2 H) 7.44 - 7.57 (m, 1 H)

### EXAMPLE 30

### (4-{5-[Butyl(2,6-dichlorobenzyl)amino]-1,3,4-thiadiazol-2-yl}phenyl)acetonitrile

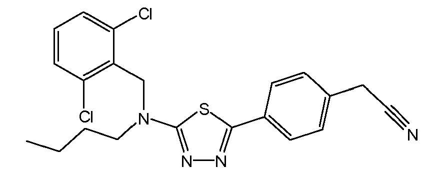

Obtained (9% yield) from 2-(bromomethyl)-1,3-dichlorobenzene and the title compound of Preparation 14 following the procedure described in example 15.
LRMS: m/z 431 (M+1)⁺
Retention time: 16.1 min (method C)
¹H NMR (200 MHz, CHLOROFORM-d) δ ppm 0.96 (t, J=7.22 Hz, 3 H) 1.31 - 1.49 (m, 2 H) 1.57 - 1.89 (m, 2 H) 2.97 - 3.41 (m, J=6.83, 6.83 Hz, 2 H) 3.74 (s, 2 H) 5.39 (s, 2 H) 6.94 - 7.36 (m, 5 H) 7.43 - 7.61 (m, 2 H)

### EXAMPLE 31

### (2S)-3-(4-{5-[Butyl(2-chlorobenzyl)amino]-1,3,4-thiadiazol-2-yl}-2,6-dimethyl-phenoxy)propane-1,2-diol

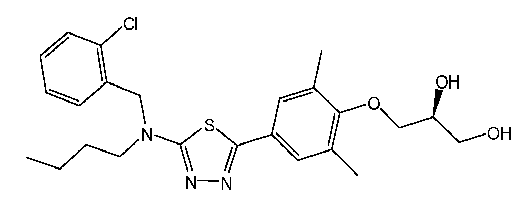

Obtained (74% yield) from the title compound of Preparation 30 following the procedure described in example 29. Reverse phase chromatography was needed for the purification in this case.
LRMS: m/z 476 (M+1)⁺
Retention time: 18.6 min (method C)
¹H NMR (200 MHz, DMSO-d₆) δ ppm 0.89 (t, J=7.42 Hz, 3 H) 1.14 - 1.43 (m, 2H) 1.50 - 1.80 (m, 2 H) 2.27 (s, 6 H) 3.38 - 3.51 (m, 4 H) 3.62 - 3.93 (m, 3 H) 4.62 (t, J=5.47 Hz, 1 H) 4.81 (s, 2 H) 4.94 (d, J=5.08 Hz, 1 H) 7.23 - 7.40 (m, 3H) 7.43 (s, 2 H) 7.46 - 7.57 (m, 1 H)

### EXAMPLE 32

### 4-{5-[Butyl(2-fluorobenzyl)amino]-1,3,4-thiadiazol-2-yl}pyridin-2-ol

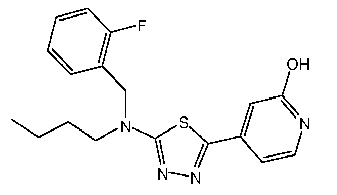

Obtained (12% yield) from the title compound of example 25 following the procedure described in Preparation 9. Reverse phase chromatography was needed for the purification in this case.
LRMS: m/z 359 (M+1)⁺
Retention time: 15.4 min (method C)
¹H NMR (200 MHz, CHLOROFORM-d) δ ppm 0.95 (t, J=7.22 Hz, 3 H) 1.10 - 1.43 (m, J=28.89 Hz, 2 H) 1.60 - 1.82 (m, 2 H) 3.24 - 3.74 (m, 2 H) 4.84 (s, 2 H) 6.55 - 6.80 (m, 1 H) 6.91 - 7.20 (m, 3 H) 7.29 - 7.51 (m, 3 H)

### EXAMPLE 33

### N-Benzyl-N-butyl-5-(3-chloro-4-methoxyphenyl)-1,3,4-thiadiazol-2-amine

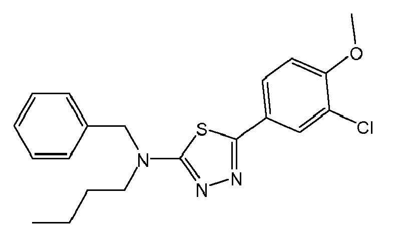

Obtained (31% yield) from (bromomethyl)benzene and the title compound of Preparation 15 following the procedure described in Example 3.
LRMS: m/z 388 (M+1)⁺
Retention time: 20.0 min (method C)
¹HNMR (200 MHz, DMSO-d₆) δ ppm 0.86 (t, J=7.22 Hz, 3 H) 1.11 - 1.40 (m, 2H) 1.50 - 1.79 (m, 2 H) 3.47 - 3.53 (m, 2 H) 3.88 (s, 3 H) 4.72 (s, 2 H) 6.93 - 7.43 (m, 6 H) 7.65 (dd, J=8.59, 1.95 Hz, 1 H) 7.73 - 7.88 (m, 1 H)

### EXAMPLE 34

### 1-Amino-3-(4-{5-[butyl(2-chlorobenzyl)amino]-1,3,4-thiadiazol-2-yl}-2,6-dimethyl-phenoxy)propan-2-ol

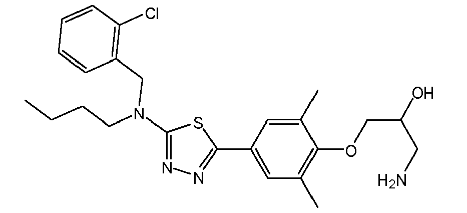

To a solution of title compound of Preparation 31 (0.19 g, 0.42 mmol) in methanol (0.8 ml), a 2M solution of ammonia in methanol (0.5 ml, 1 mmol) was added. It was stirred in a sealed tube at 60°C for 36h. It was concentrated in vacuo. The residue was dissolved in ethyl acetate and a 2N aqueous solution of HCl was added. The aqueous phase was concentrated in vacuo and finally it was liofilized to give 0.038 g (19%) of the title compound.
LRMS: m/z 475 (M+1)⁺
Retention time: 14.2 min (method C)

### EXAMPLE 35

### 1-[2-(4-{5-[Butyl(2-fluorobenzyl)amino]-1,3,4-thiadiazol-2-yl}-2,6-dimethyl-phenoxy)ethyl]azetidine-3-carboxylic acid

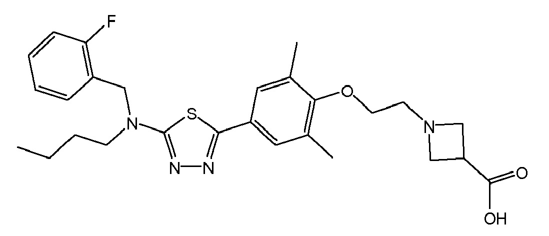

Obtained (39% yield) from azetidine-3-carboxylic acid and the title compound of Preparation 20 following the procedure described in example 22.
LRMS: m/z 513 (M+1)⁺
Retention time: 15.0 min (method C)
¹H NMR (200 MHz, DMSO-d₆) δ ppm 0.89 (t, J=7.03 Hz, 3 H) 1.08 - 1.43 (m, 2H) 1.46 - 1.78 (m, 2 H) 2.25 (s, 6 H) 2.60 - 2.85 (m, J=5.27, 5.27 Hz, 2 H) 2.97 - 3.33 (m, 3 H) 3.35 - 3.56 (m, J=5.86 Hz, 4 H) 3.53 - 3.81 (m, 2 H) 4.78 (s, 2 H) 6.98 - 7.28 (m, 2 H) 7.24 - 7.53 (m, 4 H) 8.35 (s, 1 H)

### EXAMPLE 36

### N-[2-(4-{5-[Butyl(2-fluorobenzyl)amino]-1,3,4-thiadiazol-2-yl}-2,6-dimethyl-phenoxy)ethyl]-beta-alanine

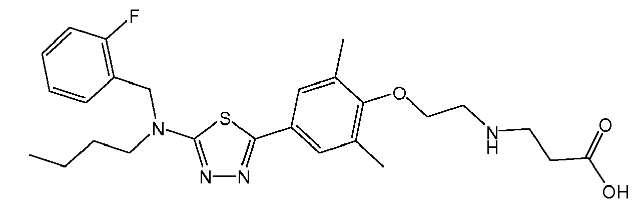

Obtained (45% yield) from 3-aminopropanoic acid and the title compound of Preparation 20 following the procedure described in example 22. In this case, the residue obtained after the concentration in vacuo of the reaction mixture was directly purified by reverse phase chromatography.
LRMS: m/z 501 (M+1)⁺
Retention time: 14.6 min (method C)
¹H NMR (200 MHz, DMSO-d₆) δ ppm 0.85-0.92 (t, 3 H) 1.16 - 1.41 (m, 2 H) 1.46 - 1.76 (m, 2 H) 2.15 - 2.31 (m, 6 H) 2.29 - 2.41 (m, 2 H) 2.74 - 2.93 (m, 2H) 2.93 - 3.12 (m, 2 H) 3.34 - 3.63 (m, 2 H) 3.74 - 4.00 (m, 2 H) 4.54 - 4.96 (m, 2H) 6.98 - 7.32 (m, 4 H) 7.27 - 7.54 (m, 2 H)

### EXAMPLE 37

### N-[2-(4-{5-[Butyl(2-fluorobenzyl)amino]-1,3,4-thiadiazol-2-yl}-2,6-dimethyl-phenoxy)ethyl]-N-methylglycine

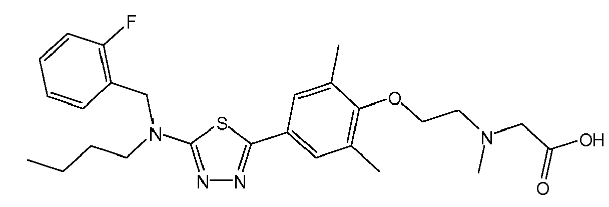

Obtained (73% yield) from 2-(methylamino)acetic acid and the title compound of Preparation 20 following the procedure described in example 36.
LRMS: m/z 501 (M+1)⁺
Retention time: 16.4 min (method C)
¹H NMR (200 MHz, DMSO-d₆) δ ppm 0.89 (t, J=7.22 Hz, 3 H) 1.17 - 1.41 (m, J=7.42 Hz, 2 H) 1.46 - 1.75 (m, 2 H) 2.27 (s, 6 H) 2.47 (s, 3 H) 3.00 (t, 2 H) 3.40 - 3.64 (m, 4 H) 3.74 - 4.00 (m, J=5.47 Hz, 2 H) 4.78 (s, 2 H) 6.88 - 7.57 (m, 6 H) 8.29 (s, 1 H)

### EXAMPLE 38

### 1-[2-(4-{5-[Butyl(2-fluorobenzyl)amino]-1,3,4-thiadiazol-2-yl}-2,6-dimethyl-phenoxy)-1-cyanoethyl]azetidine-3-carboxylic acid

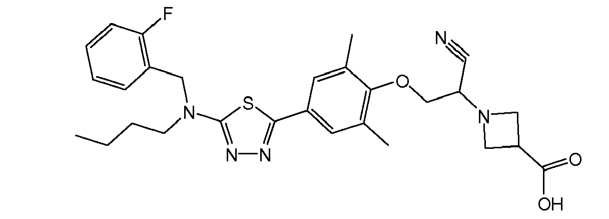

Obtained (22% yield) as a by-product from azetidine-3-carboxylic acid and the title compound of Preparation 20 following the procedure described in example 22.
LRMS: m/z 538 (M+1)⁺
Retention time: 19.1 min (method C)
¹H NMR (200 MHz, DMSO-d₆) δ ppm 0.84 (t, J=6.83 Hz, 3 H) 1.11 - 1.35 (m, 2H) 1.42 - 1.73 (m, 2 H) 2.14 (s, 6 H) 2.93 - 3.64 (m, 7 H) 3.69 - 3.91 (m, 2 H) 4.04 - 4.27 (m, 1 H) 4.73 (s, 2 H) 6.66 - 7.50 (m, 6 H)

### EXAMPLE 39

### 1-[2-(4-{5-[Butyl(2-fluorobenzyl)amino]-1,3,4-thiadiazol-2-yl}-2,6-dimethyl-phenoxy)ethyl]piperidine-4-carboxylic acid

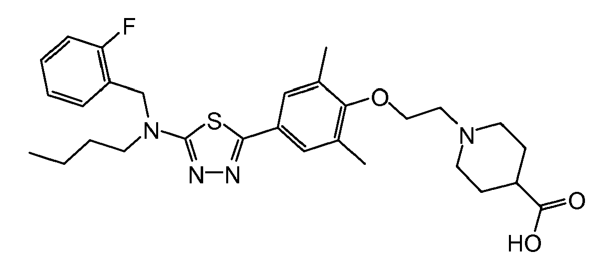

Obtained (30% yield) from piperidine-4-carboxylic acid and the title compound of Preparation 20 following the procedure described in example 22. In this case, after the concentration in vacuo, the residue was dissolved in dioxane and the suspension obtained was filtered through celite. A 4M solution of HCl in dioxane was added to the filtrate. The solid obtained was recrystallized from diethyl ether.
LRMS: m/z 541 (M+1)⁺
Retention time: 14.0 min (method C)
¹H NMR (400 MHz, DMSO-d₆) δ ppm 0.89 (t, J=7.42 Hz, 3 H) 1.21 - 1.38 (m, 2H) 1.42 - 1.67 (m, 4 H) 1.69 - 1.88 (m, J=12.90, 3.13 Hz, 2 H) 1.99 - 2.12 (m, 2H) 2.14 - 2.23 (m, 1 H) 2.27 (s, 6 H) 2.66 (t, J=5.67 Hz, 2 H) 2.81 - 2.94 (m, J=11.72 Hz, 2 H) 3.39 - 3.54 (m, 2 H) 3.86 (t, J=5.67 Hz, 2 H) 4.78 (s, 2 H) 7.11 - 7.30 (m, 2 H) 7.33 - 7.40 (m, 2 H) 7.43 (s, 2 H) 8.33 (s, 1 H)

### EXAMPLE 40

### 1-(4-{5-[Ethyl(pyridin-2-yl)amino]-1,3,4-thiadiazol-2-yl}benzyl)azetidine-3-carboxylic acid

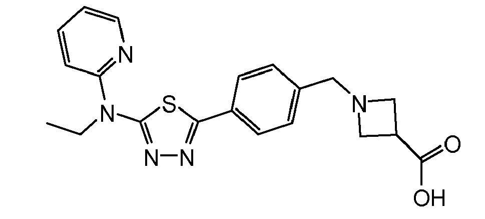

Obtained (17% yield) from azetidine-3-carboxylic acid and the title compound of Preparation 34 following the procedure described in example 22. In this case, the residue obtained after the concentration in vacuo of the reaction mixture was directly purified by reverse phase chromatography.
LRMS: m/z 396 (M+1)⁺
Retention time: 10.4 min (method C)
¹H NMR (400 MHz, METHANOL-d₄) δ ppm 1.43 (t, J=7.03 Hz, 3 H) 3.43 - 3.63 (m, 1 H) 4.11 - 4.33 (m, J=8.21 Hz, 3 H) 4.42 (s, 1 H) 4.46 - 4.62 (m, J=7.03, 7.03, 7.03 Hz, 2 H) 7.12 (dd, J=6.84, 5.28 Hz, 1 H) 7.37 (d, J=8.60 Hz, 1 H) 7.60 (d, J=8.21 Hz, 2 H) 7.82 - 7.94 (m, 1 H) 8.04 (d, J=8.21 Hz, 1 H) 8.19 (d, 1 H) 8.46 (d, J=3.91 Hz, 1 H)

### EXAMPLE 41

### 1-(4-{5-[Butyl(pyridin-2-yl)amino]-1,3,4-thiadiazol-2-yl}benzyl)azetidine-3-carboxylic acid

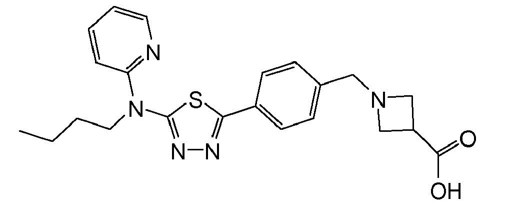

Obtained (24% yield) from azetidine-3-carboxylic acid and the title compound of Preparation 36 following the procedure described in example 22. In this case, the residue obtained after the concentration in vacuo of the reaction mixture was directly purified by reverse phase chromatography.
LRMS: m/z 424 (M+1)⁺
Retention time: 12.5 min (method C)
¹H NMR (400 MHz, METHANOL-d₄) δ ppm 0.94 (t, J=7.42 Hz, 3 H) 1.31 - 1.53 (m, 2 H) 1.66 - 1.85 (m, 2 H) 3.31 - 3.45 (m, 1 H) 4.11 (d, J=8.21 Hz, 3 H) 4.31 (s, 1 H) 4.34 - 4.41 (m, 2 H) 7.02 (dd, J=7.62, 5.28 Hz, 1 H) 7.25 (d, J=8.60 Hz, 1 H) 7.50 (d, J=8.60 Hz, 2 H) 7.70 - 7.82 (m, 1 H) 7.93 (d, J=8.21 Hz, 2 H) 8.37 (d, J=3.91 Hz, 1 H)

### EXAMPLE 42

### N-Butyl-N-[5-(4-methoxy-3,5-dimethylphenyl)-1,3,4-thiadiazol-2-yl]benzenesulfonamide

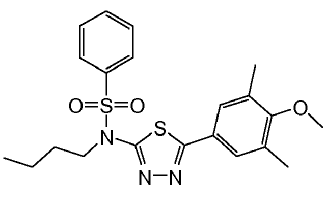

To a solution of N-butyl-5-(4-methoxy-3,5-dimethylphenyl)-1,3,4-thiadiazol-2-amine (Preparation 2) in pyridine (1 ml) under inert atmosphere DMAP (0.01 g, 0.08 mmols) and benzenesulfonyl chloride were added. The suspension obtained was stirred in the microwave at 110°C for 30 minutes (Biotage Initiator® device and high absorvance). It was poured into water and extracted with ethyl acetate. The organic phase was washed with brine, dried, filtered and concentrated in vacuo. It was purified by reverse phase chromatography to yield 0.015 g (5%) of the title compound.
LRMS: m/z 432 (M+1)⁺
Retention time: 20.23 min (method C)
1 H NMR (200 MHz, CHLOROFORM-d) δ ppm 0.93 (t, J=7.42 Hz, 3 H) 1.28 - 1.46(m,2H)1.66-1.87(m,2H)2.34(s,6H)3.76(s,3H)3.86-4.12(m,2H) 7.43 - 7.74 (m, 5 H) 7.73 - 7.94 (m, 2 H)

### EXAMPLE 43

### 1-(4-{5-[(Benzylsulfonyl)(butyl)amino]-1,3,4-thiadiazol-2-yl}benzyl)azetidine-3-carboxylic acid

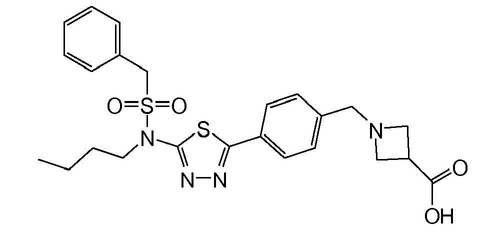

Obtained (4% yield) from azetidine-3-carboxylic acid and the title compound of Preparation 37 following the procedure described in example 22
LRMS: m/z 501 (M+1)⁺
Retention time: 13.0 min (method C)
¹H NMR (400 MHz, DMSO-d₆) δ ppm 0.88 (t, J=7.42 Hz, 3 H) 1.09 - 1.42 (m, 2 H) 1.46 - 1.82 (m, 2 H) 3.05 - 3.26 (m, 5 H) 3.60 (s, 2 H) 3.78-4.10 (m, 2 H) 4.95 (s, 2 H) 7.16 - 7.52 (m, 7 H) 7.58 - 7.89 (m, 2 H)

### EXAMPLE 44

### 1-(4-{5-[Butyl(phenylsulfonyl)amino]-1,3,4-thiadiazol-2-yl}benzyl)azetidine-3-carboxylic acid

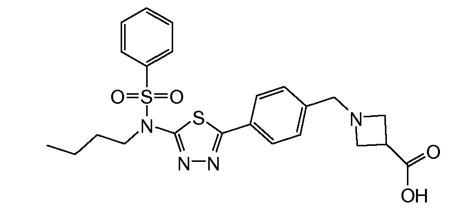

Obtained (45% yield) from azetidine-3-carboxylic acid and the title compound of Preparation 38 following the procedure described in example 22.
LRMS: m/z 487 (M+1)⁺
Retention time: 13.4 min (method C)
¹H NMR (400 MHz, CHLOROFORM-d) δ ppm 0.93 (t, J=7.42 Hz, 3 H) 1.21 - 1.46 (m, 2 H) 1.65 - 1.83 (m, 2 H) 3.23 - 3.41 (m, 1 H) 3.71 (s, 2 H) 3.84 - 3.92 (m, 1 H) 3.95 - 4.04 (m, 2 H) 4.07 - 4.19 (m, 3 H) 7.20 - 7.34 (m, 1 H) 7.47 - 7.57 (m, 3 H) 7.59 - 7.68 (m, J=7.42, 7.42 Hz, 1 H) 7.83 (dd, J=7.42 Hz, 2 H) 7.93 (d, J=8.21 Hz, 2 H)

### EXAMPLE 45

### 1-(4-{5-[(Benzylsulfonyl)(ethyl)amino]-1,3,4-thiadiazol-2-yl}benzyl)azetidine-3-carboxylic acid

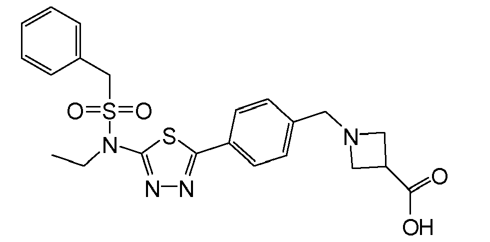

Obtained (8% yield) from azetidine-3-carboxylic acid and the title compound of Preparation 40 following the procedure described in example 22.
LRMS: m/z 473 (M+1)⁺
Retention time: 10.8 min (method C)
¹H NMR (400 MHz, DMSO-d₆) δ ppm 1.25 (t, J=7.03 Hz, 3 H) 3.20 (s, 2 H) 3.59 (m, 1 H) 4.00 (d, J=7.03 Hz, 2 H) 4.96 (s, 2 H) 7.05 - 7.53 (m, 7 H) 7.73 (d, J=8.21 Hz, 2 H)

### PHARMACOLOGICAL ACTIVITY

### 35S-GTP-g binding assay:

The effect of the compounds was measured using a 35S-GTPyS binding assay. Briefly, membranes were incubated in a buffer containing 20 mM HEPES pH 7.4, 100 mM NaCl, 10 mM MgCl2, 10µM GDP, 50µg/ml saponin and 0.2% fatty acid-free BSA at various concentrations (0.1nM-10µM) and 0.1nM 35S-GTPyS. 10µM S1P was used as 100% maximum efficacy. The assay was incubated for 90 min at room temperature with gentle mixing, and terminated by filtrating the reaction mixture through GF/C filter plates using the Manifold Filtration System. The filters were immediately washed with sodium phosphate pH 7.4 buffers. After drying the filter plate's scintillant liquid were added to each well and 35S-GTPyS binding was measured on a Trilux Scintillation Counter. The results are shown in Table 1.

**Table 1**

| **EXAMPLES** | **EC₅₀ (nM)** |
|---|---|
| 1 | 39 |
| 16 | 18 |
| 29 | 15 |
| 34 | 32 |
| 35 | 33 |
| 36 | 5 |
| 43 | 123 |

The 2-aminothiadiazole derivatives of the invention may also be combined with other active compounds in the treatment of diseases known to be susceptible to improvement by treatment with a sphingosine-1-phosphate receptor agonist (S1P1).

The combinations of the invention can optionally comprise one or more additional active substances which are known to be useful in the treatment of autoimmune diseases, chronic immune and inflammatory diseases, transplant rejection, malignant neoplastic diseases, angiogenic-related disorders, pain, neurological diseases,viral and infectious diseases, such as (a) beta interferons such as Betaseron, Avonex or Rebif, (b), immunomodulators such as glatiramer acetate, (c) inhibitors of DNA synthesis and repair, such as Mitoxantrone, (d) anti-alpha 4 integrin antibodies, such as Natalizumab (Tysabri), (e) alpha 4 integrin antagonists such as R-1295 , TBC-4746, CDP-323, ELND-002, Firategrast and TMC-2003, (f), dyhydrofolate reductase inhibitors, such as Methotrexate or CH-1504, (g) glucocorticoids such as prednisone or methylprednisolone, (h), DHODH inhibitors such as Teriflunomide, (i) fumaric acid esters, such as *BG-12*, (j) immunomodulators such as Laquinimod, (k) anti-CD20 monoclonal antibodies such as Rituximab, Ocrelizumab Ofatumumab or TRU-015, (l) anti-CD52 such as alemtuzumab, (m) anti-CD25 such as daclizumab, (n) anti-CD88, such as eculizumab or pexilizumab, (o) calcineurin inhibitors such as cyclosporine A or tacrolimus, (p) IMPDH inhibitors, such as mycophenolate mophetyl, (q) cannabinoid receptor agonists such as Sativex, (r) chemokine CCR1 antagonists such as MLN-3897 or PS-031291, (s) chemokine CCR2 antagonists such as INCB-8696, (t) interferon alpha such as Sumiferon MP, (u) NF-kappaB activation inhibitors such as FAE and MLN-0415, (v) JAK inhibitors such as CP-690550 or INCB018424, (W) Syk inhibitors, such as R-112, (x) PKC inhibitors, such as NVP-AEB071, (y) phosphosdiesterase IV inhibitors such as GRC-4039, (z) P38 Inhibitors such as ARRY-797, and (aa) MEK inhibitors, such as ARRY-142886 or ARRY-438162

The combinations of the invention may be used in the treatment of disorders which are susceptible to amelioration by sphingosine-1-phosphate receptors agonists (S1P1). Thus, the present application encompasses methods of treatment of these disorders, as well as the use of the combinations of the invention in the manufacture of a medicament for the treatment of these disorders.

Preferred examples of such disorders are multiple sclerosis, transplant rejection, systemic lupus erythematosus, asthma, psoriasis, rheumatoid arthritis, psoriatic arthritis and Crohn's disease, more preferably multiple sclerosis, transplant rejection, asthma and rheumatoid arthritis, and most preferably multiple sclerosis.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the sphingosine-1-phosphate agonist of the invention, and the other optional active compounds may be administered together in the same pharmaceutical composition or in different compositions intended for separate, simultaneous, concomitant or sequential administration by the same or a different route.

One execution of the present invention consists of a kit of parts comprising a sphingosine-1-phosphate agonist of the invention together with instructions for simultaneous, concurrent, separate or sequential use in combination with another active compound useful in the treatment of multiple sclerosis, transplant rejection, systemic lupus erythematosus, asthma, psoriasis, rheumatoid arthritis, psoriatic arthritis and Crohn's disease,

Another execution of the present invention consists of a package comprising a sphingosine-1-phosphate agonist of formula (I) and another active compound useful in the treatment of multiple sclerosis, transplant rejection, systemic lupus erythematosus, asthma, psoriasis, rheumatoid arthritis, psoriatic arthritis and Crohn's disease,

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2µg and 150 µg of each therapeutically active ingredient. Alternatively, the active ingredient (s) may be presented without excipients.

Packaging of the formulation for inhalation may be carried out by using suitable inhaler devices such as the Novolizer SD2FL which is described in the following patent applications: WO 97/000703, WO 03/000325 and WO 03/061742.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

Effective doses are normally in the range of 2-2000 mg of active ingredient per day. Daily dosage may be administered in one or more treatments, preferably from 1 to 4 treatments, per day. Preferably, the active ingredients are administered once or twice a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The following preparations forms are cited as formulation examples:

### COMPOSITION EXAMPLE 1

50,000 capsules, each containing 100 mg of N-butyl-N-(2,6-difluorobenzyl)-5-(4-methoxy-3,5-dimethylphenyl)-1,3,4-thiadiazol-2-amine (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of N-butyl-N-(2,6-difluorobenzyl)-5-(4-methoxy-3,5-dimethylphenyl)-1,3,4-thiadiazol-2-amine (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A compound of formula (I), or a pharmaceutically acceptable salt or N-oxide
thereof: 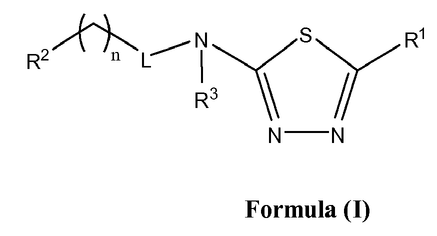 wherein:
• L represents a direct bond or a -S(O)₂- group,
• n is an integer having a value from 0 to 2,
• R¹ represents a pyridyl group or an imidazo[1,2-α]pyridinyl group, wherein the pyridyl group is substituted with one or more substituents selected from halogen atoms, a hydroxy group, a linear or branched C₁₋₄ alkyl group and a C₁₋₄ alkoxy group, or
• R¹ represents a group of formula: 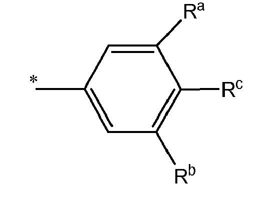 wherein:
○ R^{a} and R^{b} independently represent a hydrogen atom, a halogen atom or a linear or branched C₁₋₄ alkyl group,
○ R^{c} represents a hydroxy group, a linear or branched C₁₋₄ alkyl group or a C₁₋₄ alkoxy group wherein the alkyl and the alkoxy groups independently are optionally substituted with one or more substituents selected from a hydroxy group, a cyano group and a - NR'R" groups and wherein
■ R' represents a hydrogen atom or a linear or branched C₁₋₄ alkyl group,
■ R" represents a hydrogen atom or a linear or branched C₁₋₄ alkyl group optionally substituted with a hydroxycarbonyl group; or
■ R' and R" together with the nitrogen atom to which they are attached form a 4 to 6 membered, saturated heterocyclic ring optionally substituted with a hydroxycarbonyl group.
• R² represents a pyridyl group, a C₃₋₆ cycloalkyl group or a phenyl group which is optionally substituted with one or more substituents selected from a halogen atom, a cyano group, and a C₁₋₄ alkoxy group;
• R³ represents a C₃₋₆ cycloalkyl group, a C₃₋₆ cycloalkyl-C₁₋₂ alkyl group or a linear or branched C₂₋₄ alkyl group optionally substituted with one or more substituents selected from halogen atoms and a C₁₋₂ alkoxy group;

2. A compound according to claim 1, wherein L represents a direct bond.

3. A compound according to claim 1 or 2, wherein n has a value of 1 or 2, preferably 1.

4. A compound according to any preceding claims, wherein R¹ represents
• a pyridyl group substituted with one or two substituents selected from halogen atoms, a methyl group and a methoxy group, or
• a group of formula: 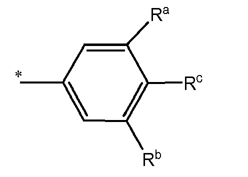 wherein:
o R^{a} and R^{b} independently represent a hydrogen atom or a linear or branched C₁₋₄ alkyl group,
o R^{c} represents a hydroxy group, a linear or branched C₁₋₄ alkyl group or a C₁₋₄ alkoxy group wherein the alkyl and the alkoxy groups independently are optionally substituted with one or more substituents selected from a hydroxy group, a cyano group and a - NR'R" group wherein
■ R' represents a hydrogen atom or a methyl group,
■ R" represents a hydrogen atom or a linear or branched C₁₋₄ alkyl group optionally substituted with a hydroxycarbonyl group; or
■ R' and R" together with the nitrogen atom to which they are attached form a 4 to 6 membered, saturated heterocyclic ring substituted with a hydroxycarbonyl group.

5. A compound according to claim 4, wherein R¹ represents:
• a pyridyl group substituted with one substituent selected from a methyl group and a methoxy group, or
• a group of formula: 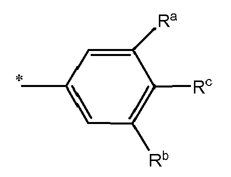 wherein:
○ both R^{a} and R^{b} represent a methyl group,
○ R^{c} represents a hydroxy group, a methoxy group or a C₂₋₄ alkoxy group substituted with one or two substituents selected from a hydroxy group, and a -NR'R" group, wherein
■ R' represents a hydrogen atom or a methyl group,
■ R" represents a hydrogen atom, a C₁₋₂ alkyl group substituted with a hydroxycarbonyl group; or
■ R' and R" together with the nitrogen atom to which they are attached form a 4 to 6 membered, saturated heterocyclic ring substituted with a hydroxycarbonyl group.

6. A compound according to any preceding claims, wherein R² represents a phenyl group which is substituted with one or two substituents selected from chlorine atoms, fluorine atoms, cyano group and methoxy group, preferably fluorine atoms, chlorine atoms and cyano group.

7. A compound according to any preceding claims, wherein R³ represents a linear or branched C₂₋₄ alkyl group optionally substituted with one or more substituents selected from a halogen atom and a C₁₋₂ alkoxy group.

8. A compound according to claim 7, wherein R³ represents a propyl or a butyl group, preferably a butyl group.

9. A compound according to any preceding claims wherein:
• L represent a direct bond,
• n has a value of 1,
• R¹ represents a pyridyl group substituted with one substituent selected from a methyl group and a methoxy group, or
• R¹ represents a group of formula: 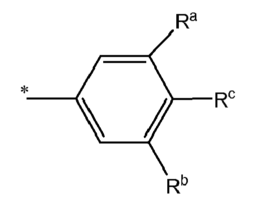 wherein:
○ both R^{a} and R^{b} represent a methyl group,
○ R^{c} represents a hydroxy group, a methoxy group or a C₂₋₄ alkoxy group substituted with one or two substituents selected from a hydroxy group, and a -NR'R" group, wherein
■ R' represents a hydrogen atom or a methyl group,
■ R" represents a hydrogen atom, a C₁₋₂ alkyl group substituted with a hydroxycarbonyl group; or
■ R' and R" together with the nitrogen atom to which they are attached form a 4 to 6 membered, saturated heterocyclic ring substituted with a hydroxycarbonyl group
• R² represents a phenyl group which is substituted with one or two substituents selected from chlorine atoms, fluorine atoms and cyano group, and
• R³ represents a butyl group.

10. A compound according to claim 1, wherein:
• R¹ represents a pyridyl group or an imidazo[1,2-α]pyridinyl group, wherein the pyridyl group is substituted with one or more substituents selected from chlorine atoms, a hydroxy group, a methyl group and a methoxy group, or
• R¹ represents a group of formula: 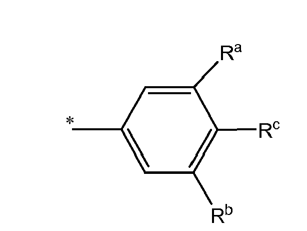 wherein:
○ R^{a} and R^{b} independently represent a hydrogen atom, a chlorine atom or a methyl group,
○ R^{c} represents a hydroxy group, a linear or branched C₁₋₄ alkyl group or C₁₋₄ alkoxy group wherein the alkyl and the alkoxy groups independently are optionally substituted with one or more substituents selected from a hydroxy group, a cyano group and a - NR'R" group and wherein
■ R' represents a hydrogen atom or a methyl group,
■ R" represents a hydrogen atom, a C₁₋₂ alkyl group optionally substituted with a hydroxycarbonyl group; or
■ R' and R" together with the nitrogen atom to which they are attached form a 4 to 6 membered, saturated heterocyclic ring optionally substituted with a hydroxycarbonyl group.
• R² represents a pyridyl group, a cyclobutyl group or a phenyl group which is optionally substituted with one or more substituents selected from a chlorine atom, a fluorine atom, a cyano group, and a methoxy group; and
■ R³ represents a cyclopentyl group, a cyclopropylmethyl group, ethyl group, isopropyl group, a propyl group, n-butyl group, 4-trifluorobutyl group or a methoxyethyl group.

11. A compound according to claim 1 which one of:
N-butyl-N-(2-chlorobenzyl)-5-(4-methoxy-3,5-dimethylphenyl)-1,3,4-thiadiazol-2-amine,
N-benzyl-N-butyl-5-(4-methoxy-3,5-dimethylphenyl)-1,3,4-thiadiazol-2-amine N-butyl-N-(2-fluorobenzyl)-5-(4-methoxy-3,5-dimethylphenyl)-1,3,4-thiadiazol-2-amine,
N-butyl-N-(2,6-difluorobenzyl)-5-(4-methoxy-3,5-dimethylphenyl)-1,3,4-thiadiazol-2-amine,
N-butyl-N-(2,4-difluorobenzyl)-5-(4-methoxy-3,5-dimethylphenyl)-1,3,4-thiadiazol-2-amine,
N-butyl-N-(2,6-dichlorobenzyl)-5-(4-methoxy-3,5-dimethylphenyl)-1,3,4-thiadiazol-2-amine,
2-({butyl[5-(4-methoxy-3,5-dimethylphenyl)-1,3,4-thiadiazol-2-yl]amino}methyl)-benzonitrile,
N-butyl-N-(3-methoxybenzyl)-5-(4-methoxy-3,5-dimethylphenyl)-1,3,4-thiadiazol-2-amine,
N-(2-chlorobenzyl)-N-(cyclopropylmethyl)-5-(4-methoxy-3,5-dimethylphenyl)-1,3,4-thiadiazol-2-amine,
N-(2-chlorobenzyl)-5-(4-methoxy-3,5-dimethylphenyl)-N-(2-methoxyethyl)-1,3,4-thiadiazol-2-amine,
N-(2-chlorobenzyl)-5-(4-methoxy-3,5-dimethylphenyl)-N-propyl-1,3,4-thiadiazol-2-amine,
N-(2-chlorobenzyl)-5-(4-methoxy-3,5-dimethylphenyl)-N-(4,4,4-trifluorobutyl)-1,3,4-thiadiazol-2-amine,
N-(2-chlorobenzyl)-N-cyclopentyl-5-(4-methoxy-3,5-dimethylphenyl)-1,3,4-thiadiazol-2-amine,
N-(2-chlorobenzyl)-N-isopropyl-5-(4-methoxy-3,5-dimethylphenyl)-1,3,4-thiadiazol-2-amine,
N-butyl-N-(2-fluorobenzyl)-5-(2-methylpyridin-4-yl)-1,3,4-thiadiazol-2-amine,
N-butyl-N-(2,6-dichlorobenzyl)-5-(2-methylpyridin-4-yl)-1,3,4-thiadiazol-2-amine,
N-butyl-N-(cyclobutylmethyl)-5-(2-methylpyridin-4-yl)-1,3,4-thiadiazol-2-amine,
N-butyl-5-(2-methylpyridin-4-yl)-N-(2-phenylethyl)-1,3,4-thiadiazol-2-amine, 5-(4-methoxy-3,5-dimethylphenyl)-N-propyl-N-(pyridin-4-ylmethyl)-1,3,4-thiadiazol-2-amine,
N-butyl-N-(2,6-dichlorobenzyl)-5-imidazo[1,2-a]pyridin-6-yl-1,3,4-thiadiazol-2-amine,
N-butyl-N-(2-chlorobenzyl)-5-imidazo[1,2-a]pyridin-6-yl-1,3,4-thiadiazol-2-amine, 1-(4-{5-[butyl(2-chlorobenzyl)amino]-1,3,4-thiadiazol-2-yl}benzyl)azetidine-3-carboxylic acid,
1-(4-{5-[butyl(2,6-dichlorobenzyl)amino]-1,3,4-thiadiazol-2-yl}benzyl)azetidine-3-carboxylic acid,
N-butyl-5-(2-chloro-6-methoxypyridin-4-yl)-N-(2-fluorobenzyl)-1,3,4-thiadiazol-2-amine,
N-butyl-N-(2-fluorobenzyl)-5-(2-methoxypyridin-4-yl)-1,3,4-thiadiazol-2-amine,
N-butyl-N-(2,6-dichlorobenzyl)-5-(6-methoxypyridin-3-yl)-1,3,4-thiadiazol-2-amine, 4-{5-[butyl(2-chlorobenzyl)amino]-1,3,4-thiadiazol-2-yl}-2,6-dimethylphenol, N-butyl-N-(2,6-dichlorobenzyl)-5-imidazo[1,2-a]pyridin-7-yl-1,3,4-thiadiazol-2-amine,
(2R)-3-(4-{5-[butyl(2-chlorobenzyl)amino]-1,3,4-thiadiazol-2-yl}-2,6-dimethyl-phenoxy)-propane-1,2-diol,
(4-{5-[butyl(2,6-dichlorobenzyl)amino]-1,3,4-thiadiazol-2-yl}phenyl)acetonitrile (2S)-3-(4-{5-[butyl(2-chlorobenzyl)amino]-1,3,4-thiadiazol-2-yl}-2,6-dimethyl-phenoxy)-propane-1,2-diol,
4-{5-[butyl(2-fluorobenzyl)amino]-1,3,4-thiadiazol-2-yl}pyridin-2-ol, N-benzyl-N-butyl-5-(3-chloro-4-methoxyphenyl)-1,3,4-thiadiazol-2-amine,
1-amino-3-(4-{5-[butyl(2-chlorobenzyl)amino]-1,3,4-thiadiazol-2-yl}-2,6-dimethyl-phenoxy)propan-2-ol,
1-[2-(4-{5-[butyl(2-fluorobenzyl)amino]-1,3,4-thiadiazol-2-yl}-2,6-dimethylphenoxy)-ethyl]azetidine-3-carboxylic acid,
N-[2-(4-{5-[butyl(2-fluorobenzyl)amino]-1,3,4-thiadiazol-2-yl}-2,6-dimethylphenoxy)-ethyl]-beta-alanine,
N-[2-(4-{5-[butyl(2-fluorobenzyl)amino]-1,3,4-thiadiazol-2-yl}-2,6-dimethylphenoxy)-ethyl]-N-methylglycine,
1-[2-(4-{5-[butyl(2-fluorobenzyl)amino]-1,3,4-thiadiazol-2-yl}-2,6-dimethylphenoxy)-1-cyanoethyl]azetidine-3-carboxylic acid,
1-[2-(4-{5-[butyl(2-fluorobenzyl)amino]-1,3,4-thiadiazol-2-yl}-2,6-dimethylphenoxy)-ethyl]piperidine-4-carboxylic acid,
1-(4-{5-[ethyl(pyridin-2-yl)amino]-1,3,4-thiadiazol-2-yl}benzyl)azetidine-3-carboxylic acid,
1-(4-{5-[butyl(pyridin-2-yl)amino]-1,3,4-thiadiazol-2-yl}benzyl)azetidine-3-carboxylic acid,
N-butyl-N-[5-(4-methoxy-3,5-dimethylphenyl)-1,3,4-thiadiazol-2-yl]benzenesulfonamide,
1-(4-{5-[(benzylsulfonyl)(butyl)amino]-1,3,4-thiadiazol-2-yl}benzyl)azetidine-3-carboxylic acid,
1-(4-{5-[butyl(phenylsulfonyl)amino]-1,3,4-thiadiazol-2-yl}benzyl)azetidine-3-carboxylic acid, and
1-(4-{5-[(benzylsulfonyl)(ethyl)amino]-1,3,4-thiadiazol-2-yl}benzyl)azetidine-3-carboxylic acid.

12. A compound according to any one of claims 1 to 11 for use in the treatment of the human or animal body by therapy.

13. A compound according to any one of claims 1 to 11 for use in the treatment of a pathological condition or disease susceptible to amelioration by sphingosine-1-phosphate receptors (S1P1) agonists.

14. A compound for use according to claim 13, wherein the pathological condition or disease is selected from autoimmune diseases, chronic immune and inflammatory diseases, transplant rejection, malignant neoplastic diseases, angiogenic-related disorders, pain, neurological diseases,viral and infectious diseases.

15. A compound for use according to claim 14 wherein the pathological condition or disease is selected from multiple sclerosis, transplant rejection, systemic lupus erythematosus, asthma, psoriasis, rheumatoid arthritis, psoriatic arthritis and Crohn's disease,

16. A pharmaceutical composition comprising a compound as defined in any one of claims 1 to 11 in association with a pharmaceutically acceptable diluent or carrier.

17. Use of a compound as defined in any one of claims 1 to 11 in the manufacture of a medicament for the treatment of a pathological condition or disease as defined in claims 13 to 15.

18. A method for treating a subject afflicted with a pathological condition or disease as defined in claims 13 to 15, which comprises administering to said subject an effective amount of a compound as defined in any one of claims 1 to 11.

19. A combination product comprising (i) a compound according to any one of claims 1 to 11; and (ii) another compound selected from:
a) Beta interferons such as Betaseron, Avonex or Rebif
b) Immunomodulators such as glatiramer acetate
c) Inhibitors of DNA synthesis and repair, such as Mitoxantrone
d) Anti-alpha 4 integrin antibodies, such as Natalizumab (Tysabri)
e) Alpha 4 integrin antagonists such as R-1295 , TBC-4746, CDP-323, ELND-002, Firategrast and TMC-2003
f) Dyhydrofolate reductase inhibitors, such as Methotrexate or CH-1504
g) Glucocorticoids such as prednisone or methylprednisolone
h) DHODH inhibitors such as teriflunomide
*i)* Fumaric acid esters, such as *BG-12*
*j)* Immunomodulators such as Laquinimod
k) Anti-CD20 monoclonal antibodies such as Rituximab, Ocrelizumab Ofatumumab or TRU-015
l) Anti-CD52 such as alemtuzumab
m) Anti-CD25 such as daclizumab
n) Anti-CD88, such as eculizumab or pexilizumab
o) Calcineurin inhibitors such as cyclosporine A or tacrolimus
p) IMPDH inhibitors, such as mycophenolate mophetyl
q) Cannabinoid receptor agonists such as Sativex
r) Chemokine CCR1 antagonists such as MLN-3897 or PS-031291
s) Chemokine CCR2 antagonists such as INCB-8696
t) Interferon alpha such as Sumiferon MP
u) NF-kappaB activation inhibitors such as FAE and MLN-0415
v) JAK inhibitors such as CP-690550 or INCB018424
w) Syk inhibitors, such as R-112
x) PKC inhibitors, such as NVP-AEB071
y) Phosphosdiesterase IV inhibitors such as GRC-4039
z) P38 Inhibitors such as ARRY-797
aa) MEK inhibitors, such as ARRY-142886 or ARRY-438162
